(19) Europäisches Patentamt
European Patent Office
Office européen des brevets

(11) **EP 1 626 777 B1**

(12) **EUROPÄISCHE PATENTSCHRIFT**

(45) Veröffentlichungstag und Bekanntmachung des Hinweises auf die Patenterteilung:
**03.10.2012 Patentblatt 2012/40**

(51) Int Cl.:
***A61N 5/06*** *(2006.01)*

(21) Anmeldenummer: 04739394.7

(86) Internationale Anmeldenummer:
**PCT/EP2004/005719**

(22) Anmeldetag: **27.05.2004**

(87) Internationale Veröffentlichungsnummer:
**WO 2004/105874 (09.12.2004 Gazette 2004/50)**

(54) **ANORDNUNG ZUR REDUKTION VON MIKROORGANISMEN**

MICRO-ORGANISM-REDUCING DEVICE

DISPOSITIF PERMETTANT DE REDUIRE LE NOMBRE DE MICRO-ORGANISMES

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.05.2003 DE 10324644**
**23.10.2003 DE 10349710**

(43) Veröffentlichungstag der Anmeldung:
**22.02.2006 Patentblatt 2006/08**

(60) Teilanmeldung:
**10003440.4 / 2 204 218**

(73) Patentinhaber: **bredent medical GmbH & Co. KG**
**89250 Senden (DE)**

(72) Erfinder:
• **VIZETHUM, Freimut**
**68199 Mannheim (DE)**
• **SCHÜTZE, Reinhold**
**A-4800 Attnang-Puchheim (AT)**

(74) Vertreter: **Schmitt, Meinrad**
**Reble & Klose**
**Rechts- und Patentanwälte**
**Konrad-Zuse-Ring 32**
**68163 Mannheim (DE)**

(56) Entgegenhaltungen:
EP-A- 0 761 257      WO-A-02/13712
WO-A-95/05214      DE-A- 3 918 965
DE-A- 10 049 999

**Beschreibung**

Die Erfindung bezieht sich auf eine Anordnung zur Reduktion von Mikroorganismen gemäß der im Oberbegriff des Patentanspruchs 1 angegebenen Merkmalen.

Aus der DE 100 49 999 A1 ist eine derartige Anordnung zur photodynamischen Therapie bekannt, in welcher eine Diagnostikleuchte und eine Therapieleuchte vereint sind. Es handelt sich um ein Tischgerät mit einem Lichtaustritt und einem an diesen anschließbaren Glasfaserlichtleiter, auf dessen distales Ende ein Vorsatz als Blendschutz für Patient und Bedienpersonal aufgesteckt werden kann. Das zur Therapie verwendete Licht wird mittels einer 150W Projektorleuchte erzeugt und durch dichriotische Filter auf eine schmale Bande zwischen 610 nm und 740 nm eingeschränkt.

Aus der WO 01 /87416 A1 sind eine Anordnung und ein Verfahren zur Reduktion bzw. Zerstörung von Mikroorganismen, wie Bakterien, unter Einsatz einer lichtaktivierbaren Substanz, nach der photodynamischen Therapie (PDT) bekannt. Mit Hilfe der lichtaktivierbaren Substanz, insbesondere eines Farbstoffes, werden die Mikroorganismen, sensibilisiert und/oder angefärbt, und nach Bestrahlung mit Licht geeigneter Wellenlänge und Energiedichte abgetötet. Das Wirkungsprinzip der PDT beruht, nach der selektiven Einwirkung und/oder Anfärbung der Mikroorganismen auf der physikalischen Wirkung der Energieübertragung auf die lichtaktivierbare Substanz, welche auch als Photosensitizer oder Photosensibilisator bezeichnet wird. Von dort kann die Energie für Reaktionen an der Zellmembran zur Verfügung gestellt werden. Die mittels eines Bestrahlungsgeräts, insbesondere eines Lasergeräts, erzeugte Energie wird damit auf die Mikroorganismen konzentriert und die Gleichgewichtslage von Reaktionen, welche auch im nicht belichteten Zustand im "normalen" Milieu ablaufen, werden verschoben und in der Folge werden die Mikroorganismen zerstört.

Ferner ist aus der EP 0 637 976 B1 die Verwendung einer lichtsensibilisierenden Substanz oder Verbindung bzw. eines Photosensitizers oder Photosensibilisators (PS) bei der Herstellung eines Medikamentes zur Verwendung bei der Desinfizierung oder Sterilisierung von Geweben der Mundhöhle oder einer Wunde oder Läsion in der Mundhöhle durch Zerstören von mit einer Erkrankung verbundenen Mikroben in einer periodontalen Tasche, in der Region zwischen dem Zahn und dem Zahnfleisch bekannt. Hierbei erfolgt eine Kontaktierung der Gewebe, der Wunde oder der Läsion mit dem Photosensitizer, wobei die mit einer Erkrankung verbundenen Mikroben den Photosensitizer aufnehmen. Es wird eine Bestrahlung der Gewebe, der Wunde oder der Läsion mit Laserlicht bei einer durch den Photosensitizer absorbierenden Wellenlänge durchgeführt. Die Keimreduktion dieser kombinierten Farbstoff- und Laserbehandlung wird für verschiedene Keime und Photosensitizer in Form von Lösungen mit unter anderem Methylenblau und Toluidinblau in unterschiedlichen, recht geringen Konzentrationen beschrieben, und zwar von 0,01 bis 0,00125 % (Gewicht pro Volumen), wobei ferner der Einfluß der applizierten Energiedichte aufgezeigt wird. Als Lichtquellen werden HeNe-Laser mit einer Wellenlänge von 634 nm und einer Leistung von 7,3 mW sowie GaAs-Laser mit einer Wellenlänge von 660 nm und einer Leistung von 11 mW verwendet.

Die DE 39 18 965 A1 betrifft ein Laserbehandlungsgerät. Laserstrahlen von einer Laserstrahlquelle werden über einen Lichtleiter zu einem Spitzenteil geführt, aus welchem das Licht zur photochemischen Behandlung austritt. Das nahe Ende des Lichtleiters kann mittels eines optischen Verbinders mit der Laserstrahlquelle verbunden werden. Das Spitzenteil ist über einen verschweißten Abschnitt mit der planen Endfläche des Lichtleiters verbunden.

In der WO 95/05214 A ist ein implantierbares Gerät für die lichtaktivierte Therapie dargestellt. Als Lichtquellen finden in verschiedenen Ausführungsformen Leuchtdioden und Festkörperlaserdioden Verwendung. Das Licht einer sich entfernt vom zu therapierenden Bereich befindenden Lichtquelle kann dem Bereich mittels eines Lichtleiters zugeführt werden. Insbesondere kann der Lichtleiter eine optische Kunststofffaser mit einer numerischen Apertur von 0,6 sein. Die Lichtleiter enden in implantierbaren Lichtdiffusoren.

Des Weiteren werden in der EP 761 257 A2 verschiedene Probeköpfe für die photodynamische Therapie in Form von Diffusoren mit verschiedenen Geometrien und Schutzummantelungen beschrieben. Die Diffusoren werden von innen durch aus einem Ende eines Lichtleiters austretendes Licht beleuchtet.

Die WO 02/13712 A2 betrifft Lichtdiffusoren für die photodynamische Therapie, welche Lichtleiter umfassen, an deren Enden ein derart aufgerauhter Abschnitt vorhanden ist, dass Licht gleichmäßig aus diesem Abschnitt in einer im Wesentlichen zylindrischen Abstrahlungscharakteristik austritt.

Hiervon ausgehend liegt der Erfindung die Aufgabe zugrunde, die Anordnung dahingehend auszubilden, dass eine effektive und steuerbare Therapie mit einem geringen apparativen Aufwand und bei einfacher Handhabung erreicht wird. Die Therapie lokaler, oberflächlicher Infektionen, vor allem im Mund-, Kiefer- und Gesichtsbereich soll mit einem geringen Aufwand und hoher Funktionalität erfolgen können. Des Weiteren soll eine möglichst homogene Bestrahlung des zu therapierenden Bereiches, insbesondere der Mundschleimhautoberfläche, erreicht werden. Im Hinblick auf die große Verbreitung und der großen Häufigkeit von Infektionen, gerade im Mund-, Kiefer- und Gesichtsbereich einschließlich dentogener Infektionen, sollen die bisher insoweit bestehenden Probleme vermieden oder zumindest verringert werden.

Die Lösung dieser Aufgabe erfolgt gemäß den im Patentanspruch 1 angegebenen Merkmalen.

Die erfindungsgemäße Anordnung ermöglicht bei einfachem Aufbau und einfacher Handhabung eine funktionssichere und praxisgerechte Anwendung der Therapie mittels einer lichtaktivierbaren Substanz und einem Bestrahlungsgerät. Die lichtaktivierbare Substanz wird in Lösung in hoher Konzentration bereitgestellt, zweckmäßig abgefüllt in einer Spritze sterilisiert und gebrauchsfertig. Vorteilhaft ist die Konzentration des Photosensitizers in einem Lösungsmittel wie wässrige Lösung oder Alkohol oder Ethanol, mit einem hohen Wert vorgegeben. Die Konzentration, und zwar Gewicht pro Volumen, ist in vorteilhafter Weise größer als 0,1%, zweckmäßig größer als 0,5%, vorgegeben, wobei als Obergrenze vorteilhaft 10%, zweckmäßig 5%, insbesondere 3% vorgegeben ist. Insbesondere hat sich eine Konzentration von zumindest näherungsweise 1% als besonders geeignet erwiesen. Das Bestrahlungsgerät, welches insbesondere als Lasergerät ausgebildet ist, ist kombiniert mit einem Applikationssystem, wobei Applikatoren mit dem Bestrahlungsgerät bevorzugt lösbar verbindbar sind. Die Applikatoren sind in vorteilhafter Weise Einmaloptiken, mittels welchen eine gezielte und exakte Bestrahlung des zu therapierenden Bereiches ermöglicht wird. Die Applikatoren werden nur einmalig zur Behandlung benutzt, so dass insbesondere den Hygieneanforderungen entsprochen wird und eine unerwünschte Übertragung von Mikroorganismen sicher vermieden wird, ohne aufwendige Maßnahmen zur etwaigen nachträglichen oder wiederholten Sterilisierung. Die Applikatoren enthalten Lichtleiter, insbesondere Faserlichtleiter, und ermöglichen problemlos intraoral die Lichtverteilung bzw. Bestrahlung und können als Pocketsonden oder Flächensonden ausgebildet sein. In bevorzugter Weise sind das Bestahlungsgerät und der wenigstens eine Applikator dahingehend ausgebildet, dass eine direkte Einkopplung des Lichtes der Lichtquelle in den Lichtleiter erfolgt. In einer besonderen Ausgestaltung der Erfindung wird ein Lichtleiter bzw. eine Lichtleiterfaser mit hoher numerischer Apertur verwendet, wobei die numerische Apertur bevorzugt größer als 0,5, insbesondere größer als 0,7 ist. Hierdurch entstehen bei der Einkopplung des Lichtes in den Applikator bzw. die Lichtleiter geringe Verluste und gleichzeitig wird gewährleistet, dass der aus dem Applikator bzw. dem Lichtleiter austretende Lichtstrahl stark öffnet.

In einer besonderen Ausgestaltung der Erfindung ist mit dem Bestrahlungsgerät eine Sperreinrichtung derart kombiniert, dass nur bei angekoppeltem Applikator und/oder Lichtleiter aus dem Bestrahlungsgerät Licht austreten kann. Solange der Applikator nicht ordnungsgemäß mit dem Bestrahlungsgerät verbunden ist, wird mittels der Sperreinrichtung der direkte Lichtaustritt aus dem Bestrahlungsgerät unterbunden. In einer besonderen Ausgestaltung enthält das Bestrahlungsgerät, insbesondere dessen Kopfteil, eine bevorzugt zentrale Bohrung, in welche das Lichtleiterende des Applikators eingeführt und fixiert wird. Die Sperreinrichtung ist insbesondere im Strahlengang des Lichtes der Lichtquelle und dem freien Ende und/oder der freien Stirnfläche des Lichtleiterendes angeordnet. Erfindungsgemäß wird die Sperreinrichtung beim Ankoppeln des Applikators und/oder beim Einführen des Lichtleiterendes in die genannte Bohrung derart, insbesondere mittels des Lichtleiterendes, betätigt, dass der Strahlengang freigegeben ist. Die genannte Bohrung und/oder das eingeschobene Lichtleiterende sind bezüglich der Lichtquelle derart angeordnet und/oder ausgerichtet, dass das Licht der Lichtquelle auf die freie Stirnfläche des Lichtleiterendes fällt, gegebenenfalls fokussiert mittels eines optischen Systems. Die Applikatoren enthalten eine Verbindungs- bzw. Steckvorrichtung, insbesondere in Form eines Luersteckers, zur Aufnahme an einem Kopf oder Kopfteil des Bestrahlungsgerätes. Ferner sind die Applikatoren in vorteilhafter Weise zumindest teilweise gebogen derart ausgebildet, dass eine gezielte Bestrahlung der zu therapierenden Bereiche, insbesondere in der Mundhöhle, ermöglicht wird. Ferner sind die Applikatoren bevorzugt zumindest teilweise flexibel ausgebildet, so dass unerwünschte Verletzungen vermieden werden.

In einer bevorzugten Ausgestaltung weist der Lichtleiter eine definierte Geometrie des Lichtaustrittsbereiches derart auf, dass der Lichtaustritt an die Form der zu belichtenden Areale des zu therapierenden Bereiches angepasst ist, wobei entweder ein flächiger oder körperhafter, dreidimensionaler Abstrahlungsbereich erzeugt wird. Ferner weist der Applikator und/oder der Lichtleiter an der Spitze einen Abstandshalter auf, mit welchem ein aktiver Lichtkreis des austretenden Lichtes angezeigt und/oder der richtige bzw. vorgegebene Belichtungsabstand festgelegt wird. Gemäß einer weiteren Ausgestaltung weist der Lichtleiter eine derartige Geometrie auf, dass das Eindringen in enge, komplex geformte Körperhöhlen und/oder Gewebstaschen ermöglicht wird und/oder diese sanft geöffnet werden können. Vorteilhaft weist der Lichtleiter eine konisch geformte Spitze auf, und zwar zweckmäßig mit einem Winkel von 1,5 bis 4° zur Senkrechten. Darüber hinaus hat es sich als besonders vorteilhaft erwiesen, den Lichtleiter im Bereich der Spitze mit einer Lichtaustrittsfläche vorgegebener Mikrostruktur im Bereich von $10\mu m$ bis $200\mu m$ zu versehen. In bevorzugter Weise weist die Spitze des Lichtleiters eine Mikrorauhigkeit mit einem Ra-Wert im Bereich von 10 bis $40\mu m$, vorzugsweise 20 bis 30 $\mu m$ auf. Des Weiteren ist der Verbindungskörper des Applikators als ein Steck- und/oder Schraubverschluß mit einem integrierten Tiefenanschlag ausgebildet, wodurch eine definierte Positionierung des in das Bestrahlungsgerät eingeführten Lichtleiterendes bezüglich der Lichtquelle in axialer Richtung gewährleistet wird.

Für die erfindungsgemäße Verwendung der Anordnung wird die lichtaktivierbare Substanz, welche bevorzugt Farbstoff enthält, zunächst in hoher Konzentration auf den zu therapierenden Bereich aufgebracht und nachfolgend wird eine Spülung mit einem Medium, insbesondere Wasser und/oder mit einem möglichst basischen pH-Wert durchgeführt. Nachfolgend wird die Bestrahlung mittels des Lichts des Bestrahlungsgeräts durchgeführt, wobei in bevorzugter Weise eine optimierte Zellschädigung erfolgt. Es hat sich als besonders effektiv erwiesen, die lichtaktivierbare Substanz zunächst in hoher Konzentration auf den zu therapierenden Bereich aufzubringen und nachfolgend eine Spülung mit einem Medium, insbesondere Wasser, und/oder mit einem möglichst hohem Sauerstoffpartialdruck durchzuführen und schließlich die Bestrahlung mittels des Lichts der genannten Lichtquelle durchzuführen, wobei bevorzugt eine optimierte Zellschädigung erfolgt. Des Weiteren hat es sich als besonders zweckmäßig erwiesen, dass nach dem Aufbringen der lichtaktivierbaren Substanz in hoher Konzentration auf den zu therapierenden Bereich und ferner vor der Belichtung mittels des Lichts der Lichtquelle eine Verringerung der Menge der lichtaktiven Substanz durchgeführt wird, und zwar insbesondere durch Abwischen und/oder Abtupfen und/oder Absaugen und/oder Abblasen.

Weiterbildungen und besondere Ausgestaltungen der Erfindung sind in den Unteransprüchen angegeben. Nachfolgend wird der Aufbau der Anordnung und deren erfindungsgemäße Verwendung im einzelnen erläutert. Die Anordnung enthält:

1. Lichtaktivierbare Substanz:

Die in Lösung vorliegende lichtaktivierbare Substanz, zum Beispiel Methylenblau, welche bevorzugt einen Farbstoff enthält und als Photosensitizer oder Photosensibilisator bezeichnet wird, ist bevorzugt in eine Spritze abgefüllt und ist sterilisiert und gebrauchsfertig. Zum Aufbringen auf den zu therapierenden Bereich ist insbesondere eine Kanüle 26g vorgesehen, welche insbesondere einen Aussendurchmesser von 0,45mm, eine Länge von 25 bis 40 mm aufweist und insbesondere um 30 bis 45 Grad abgewinkelt und elastisch ausgebildet ist.

2. Bestrahlungsgerät mit Lichtquelle, insbesondere Lasergerät oder Therapielaser, bevorzugt in folgenden Ausführungsformen:

a. Mit optischem System, insbesondere Linsenpaket, und bevorzugt aufschraubbarem Ansatz zur Lichtleiterankopplung.
b. Mit direkter Strahleinkopplung ohne Linsenpaket, mit Lichtraum vor der Diode, so dass ohne angekoppelten Lichtleiter nur wenig Licht diffus aus dem zur Kopplung des Lichtleiters vorgesehenen Zugang fällt. Die Anordnung ist ferner bevorzugt derart, dass bei Verwendung einer Diode mit monitoring das rückgestrahlte Licht dazu führt, dass die Diode abgeregelt wird.

Das Bestrahlungsgerät enthält bevorzugt eine Sperreinrichtung, mittels welcher ein Lichtaustritt unterbunden wird so lange mit dem Bestrahlungsgerät ein Applikator nicht verbunden ist.

3. Applikationssystem

Einmaloptiken oder Applikatoren, welche insbesondere jeweils nur einmal zum Einsatz gelangen und bevorzugt Lichtleiter und Kunststoffummantelung aufweisen. Diese sind bevorzugt flexibel und/oder sterilisiert und/oder gebrauchsfertig und/oder für beide vorstehend genannte Bestrahlungsgeräte kompatibel. Als Material des oder der Lichtleiter ist Glas oder Kunststoff vorgesehen, insbesondere mit großer numerischer Apertur von bevorzugt über 0,5, um möglichst viel Licht einzukoppeln und außerdem das Licht in einem großen Bereich abzustrahlen. Es ist von besonderer Bedeutung, dass aufgrund der lösbaren Verbindung zwischen dem Bestrahlungsgerät und den Applikatoren, der jeweilige Applikator im Rahmen der Erfindung als vergleichsweise einfach aufgebaute und kostengünstige Komponente und als "Wegwerfprodukt" nur einmal zur Verwendung gelangt und danach entsorgt wird. Zweckmäßig sind zwei Ausführungsformen der Applikatoren vorgesehen:

a. Pocketsonde mit konischem Abstrahlungsbereich insbesondere zum Belichten der Parodontaltasche. In einem Schritt wird die Tasche geöffnet, Gewebe beiseite geschoben und der Bereich belichtet mit Abstrahlung nach vorne und zirkulär. Die Oberfläche ist aufgerauht, um die Abstrahlung des Lichtes diffus zu erreichen (beispielsweise mit Sandpapier 100 geschliffen).

b. Flächensonde zur Belichtung oberflächlicher Areale bevorzugt mit Abstandshalter,

i. dessen Länge den richtigen Abstand zum Gewebe markiert,
ii. dessen Winkel den Bereich markiert, in dem die therapeutisch notwendigen Lichtleistungen aufgebracht werden, wobei die überlappende Bestrahlung eines Bereiches erleichtert wird.

4. Ferner in einer besonderen Weiterbildung einen TherapieController und/oder ein Programm für den PC und/oder eine eigenständige Anzeige und Kontrolleinheit. Diese dienen zur Steuerung und Orientierung des Behandlers während der Therapie. Hiermit wird vor allem die Wahl der Größe der zu belichtenden Fläche oder der Anzahl der Zähne ermöglicht und insbesondere anzeigt:

a. Die Zeit der Einwirkung des Photosensitizers,
b. die Zeit für die Spülung des Areals,

c. die Zeit für die Belichtung je cm2 oder Zahn mit akustischem Hinweis auf das Ende der Belichtung je Zahn oder cm2,

d. das Ende der Behandlung.

Die Komponenten der Anordnung werden nachfolgend erläutert:

Die Energiequelle bzw. Lichtquelle des Bestrahlungsgerätes ist derart ausgebildet, dass im relevanten Wellenlängebereich eine ausreichend hohe Penetration des Lichtes im Gewebe stattfindet, da langwellige Strahlung tiefer ins Gewebe eindringt als kurzwellige. Im Bereich des Absorptionsmaximum der lichtaktivierbaren Substanz, beispielsweise von Methylenbau (664 nm in NaCl oder 655 nm in 96% Ethanol), findet eine ausreichend tiefe Durchdringung des Gewebes mit Licht statt. Im sogenannten optischen Fenster zwischen 600-900 nm wird das Licht sehr gering von Chromophoren wie Hämoglobin oder Melanin absorbiert.

Als Energie- bzw. Lichtquellen sind vor allem Lasersysteme geeignet. Der Laser (Light Amplifikation by Stimulated Emission of Radiation) ist eine Lichtquelle, die monochromatisches, kohärentes und kollimiertes Licht mit hoher Leistung abgeben kann. Unter kohärentem Licht versteht man den sowohl zeitlich als auch räumlich phasengleich verlaufende Wellenzüge.

Im Bereich der erfindungsgemäß vorgegebenen niedrigen Leistungs- bzw. Energiedichten (0,1-100 mW/cm2) sind im Wesentlichen photochemische Prozesse wirksam. Hierbei führt die Absorption von Licht primär nicht zu einer Erwärmung des Gewebes. Diese durch athermische Laserapplikation erzeugten Effekte in biologischen Materialen werden als "laserinduzierte Biostimulation" bezeichnet. Unter Einsatz des Photosensitizers, welcher nachfolgend auch als Photosensibilisator bezeichnet wird, werden derartige Laser als Lichtquelle für die Photodynamische Therapie (PDT) benutzt. Auch bei diesen Lasern können mit höherer Leistungs- bzw. Energiedichte photothermisch induzierte Effekte auftreten.

Bei Dioden-Lasern werden Halbleiterkristalle als aktives Medium verwendet, die bei Anregung kohärente Strahlung im VIS- oder IR-Bereich emittieren. Bei diesen Lasern werden direkt durch elektrischen Strom Photonen erzeugt.

Im Zusammenhang mit dem bevorzugt zum Einsatz gelangenden Photosensitizer wird als Bestrahlungsgerät ein spezieller Diodenlaser eingesetzt, der nachfolgend als HELBO TheraLite bezeichnet wird. Der Diodenlaser HELBO TheraLite ist insbesondere für Methylen Blau geeignet.

Dieses Lasersystem ist durch die folgende Eigenschaften gekennzeichnet:

| | |
|---|---|
| • Lichtquelle | Diode |
| • Wellenlänge | 660 nm (+5) |
| • Leistung | max. 100 mW |
| • Mode | Cw bzw. kontinuierlich |
| • Lichtausgangsleistung | >40mW<50 mW |
| • Kühlungssystem | Luft |
| • Energieversorgung | Batterie oder Akku |

Die Übertragung der Licht- bzw. Laserstrahlung wird erfindungsgemäß durch das Applikationssystem ermöglicht. Das Applikationssystem sorgt für die gewünschte Strahlgeometrie am Applikationsort und ermöglicht nicht zuletzt die einfache Handhabung der Laserstrahlung zur Therapie. Teil des Applikationssystems ist die Lichtleitfaser.

Unter der Zielsetzung einer effektiven und steuerbaren Therapie in der Mundhöhle ist eine möglichst homogene Bestrahlung der Mundschleimhautoberfläche erreicht. Die Mundhöhle zeichnet sich jedoch durch eine komplexe Geometrie und durch die Anwesenheit sehr unterschiedlich absorbierender Strukturen wie Knochen, Zähne und Schleimhaut aus, die deutlich von einer planen Geometrie abweichen. Dies wird mit den erfindungsgemäßen Applikatoren gewährleistet.

Lichtleitfasersysteme können die benötigte Energie auch in Areale mit komplexem Zugang wie in der Mundhöhle leiten. Zur Einkopplung in eine Lichtleitfaser wird der Primärstrahl des Lasers im Bestrahlungsgerät entweder direkt oder über ein Linsenpacket auf das Faserende fokussiert. Die numerische Apertur der Faser bestimmt den Einkoppelwinkel derart, dass die Strahlung weitgehend in den Lichtleiter eintritt.

Die Abstrahldivergenz der Lichtleitfaser ist durch die Art der Einkopplung in die Faser Kopf und des Bestrahlungsgerätes ebenfalls durch die numerische Apertur (Sinus des Öffnungswinkels) der Faser selbst bestimmt. Eine höhere Divergenz ermöglicht einen breiteren Abstrahlwinkel.

Falls Fasern mit einem starkem Abfall der Energie bzw. bei stark schwankender Lichtverteilung über die bestrahlten Fläche verwendet werden, wird das Belichtungsfeld häufig überlappend bestrahlt werden.

Im Vergleich zur Bare fiber verfügt die bevorzugt vorgesehene Microlensfaser über ein weitestgehend homogenes Bestrahlungsprofil. Mit einer optimierten Microlensfaser lässt sich eine Leistungsverteilung über die gesamte bestrahlte Fläche mit einer Homogenität von etwa 96 % erreichen. Das Überlappen der Strahlungsfelder ist bei Verwendung einer Microlensfaser nicht erforderlich, sodass die Möglichkeit gegeben ist höchst effizient den infizierten Bereich abzudecken und unnötige Überlappungsbereiche nicht gegeben sind.

Der Applikationsmodus (extraoral oder intraoral) hängt von der Fokusgröße ab, der entsprechend der Ausdehnung des Befundes gewählt wird.

Eine bevorzugte Alternative stellen erfindungsgemäß Fasern mit einer sehr hohen numerischen Apertur dar, mit denen ebenfalls gleichmäßig ausgeleuchtete Areale erzeugt werden können.

Dabei ist erfindungsgemäß eine numerische Apertur von mindestens 0,5, vorzugsweise 0,7 oder höher vorgegeben, um aufwändiges Schleifen der Faserspitzen zu vermeiden und für die Bestrahlung von intraoralen Bereichen einen klinisch sinnvollen Abstand von 0,5 bis 1 cm einhalten zu können.

Im Rahmen der Erfindung wird insbesondere folgendes Applikationssystem mit den Applikatoren angewendet, welche als Pocketsonde und/oder Flächensonde ausgebildet sind. Diese Applikatoren enthalten Kunststofflichtleiter, die in einer Ummantelung eingebettet sind, eine Einkoppelfläche mit Anschluss an das Bestrahlungsgerät, insbesondere Lasergerät, und einen spezifisch geschliffenen und/oder eine Mikrostruktur aufweisenden Abstrahlbereich besitzen.

Die Erfindung wird nachfolgend anhand der besonderen in der Zeichnung dargestellten Ausführungsbeispiele näher erläutert, ohne dass insoweit eine Beschränkung erfolgt.

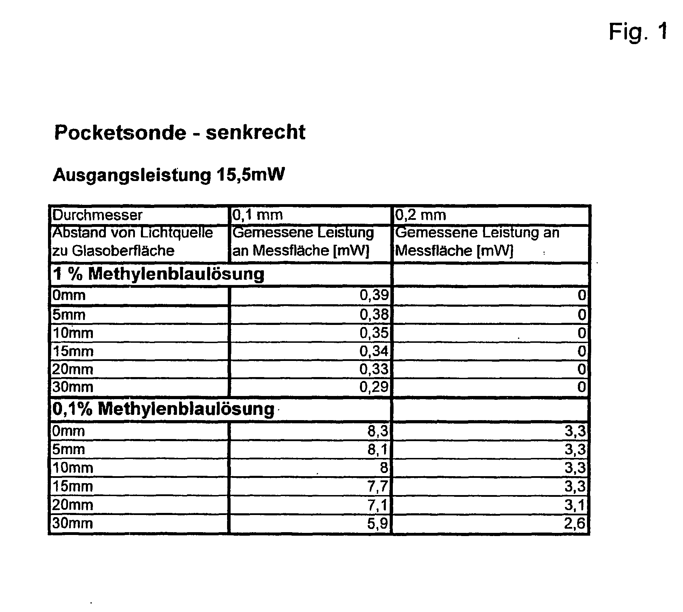

Fig. 1 enthält eine Tabelle von Messergebnissen einer Pocketsonde, welche senkrecht zu einer Glasoberfläche angeordnet war bei einer Ausgangsleistung von 15,5 mW. Hierbei sind in Abhängigkeit des Durchmessers einer Lichtleitfaser unter Berücksichtigung des Abstandes von der Lichtquelle die gemessenen Leistungen in mW angegeben. Es sei hier festgehalten, dass in bevorzugter Weise ein Photosensitizer mit hoher Konzentration, und zwar bevorzugt größer als 0,1%, insbesondere in der Größenordnung von 1 % in einem Lösungsmittel zur Verwendung gelangt, wobei gemäß Fig. 1 als Photosensitizer Methylenblau in Lösung vorgesehen ist.

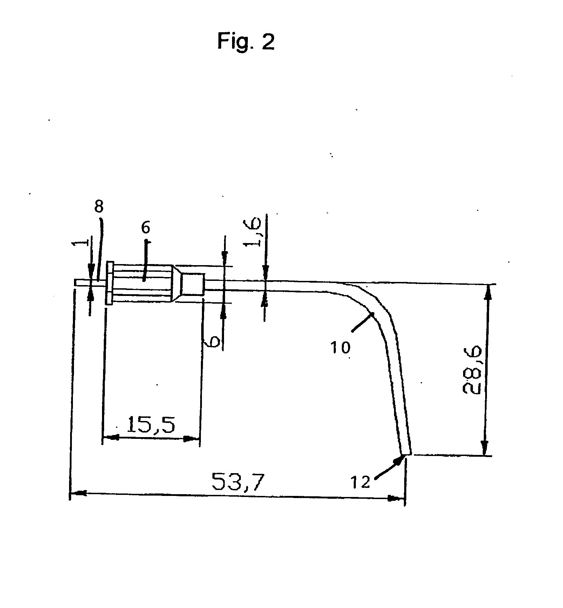

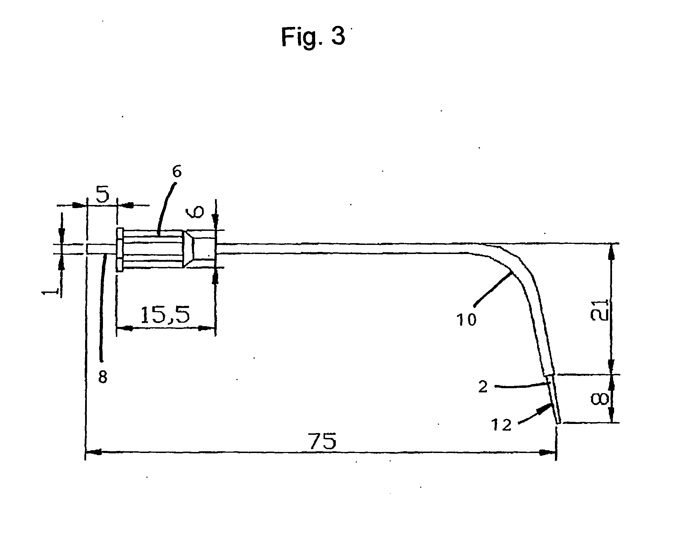

In Fig. 2 bzw. 3 sind als Applikatoren eine Pocketsonde und Flächensonde dargestellt. Es versteht sich, dass die hier ebenso wie in den weiteren Figuren beispielshaft angegebenen Maße in Millimeter bedarfsweise abgeändert werden können. Die Applikatoren enthalten einen als Faser ausgebildeten Lichtleiter 2, welcher größtenteils außen von einem Schutzmantel 4 umgeben ist. Die Applikatoren weisen ferner einen Verbindungskörper 6 auf, welcher bevorzugt als Luerstecker ausgebildet ist und über welchen die Verbindung bzw. Aufnahme im Kopf des Bestrahlungsgerätes erfolgt. Der Lichtleiter 2 ist durch den Verbindungskörper 6 hindurchgeführt und steht über diesen erfindungsgemäß mit seinem Ende 8 mit einer vorgegebenen Länge hinaus, und zwar gemäß Fig. 2 um 5 mm. Das Lichtleiterende 8 ist nicht mit einem Schutzmantel versehen und wird beim Ankoppeln des Applikators in den Kopfteil des Bestrahlungsgeräts eingeführt und in diesem positioniert. Der Lichtleiter 2 ist zumindest teilweise flexibel ausgebildet und/oder enthält einen gebogenen Bereich 10. Im übrigen sind die Abmessungen der Applikatoren derart vorgegeben, dass diese problemlos in die Mundhöhle eingeführt werden können.

Die Laserstrahlung tritt bei der in beiden Applikatoren verwendeten Laser mit 1 mm Kerndurchmesser aufgrund der Materialeigenschaften und der Anschliffgeometrie im Bereich der Spitze 12 unter einem Divergenzwinkel von im Bereich von 30 bis 60°, bevorzugt 40 bis 55 Grad, nach frontal oder insbesondere bei der Pocketsonde in einem Bereich unter von 220 bis 300 Grad, bevorzugt 240 bis 290°, insbesondere 260 bis 280 Grad zirkulär aus.

Diese Eigenschaften machen die Applikatoren zu einfach anwendbaren optischen Werkzeugen für die präzise Bestrahlung von einfachen, aber auch von komplex geformten Oberflächen.

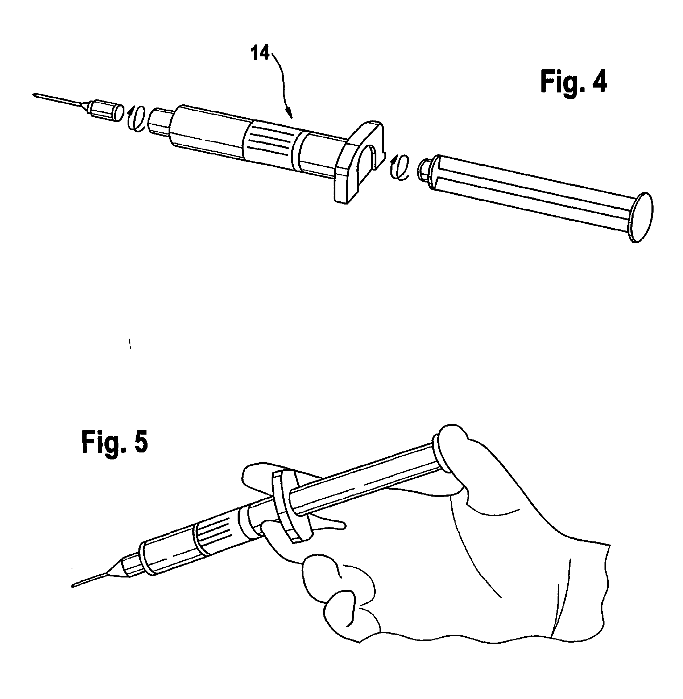

Der Aufbau und die Montage der Applikationsspitze 14, welche die lichtaktivierbare Substanz in Lösung enthält, sind in Fig. 4 und 5 dargestellt. Die die lichtaktivierbare Substanz in Lösung enthaltende Spritze 14 wird gebrauchsfertig geliefert. Vor der Anwendung muß die Verschlusskappe durch vorsichtiges Drehen von der Spitze entfernt und die beigepackte sterile Kanüle auf dem Luer Lock Adapter der Spritze fixiert werden. Auf die richtige Fingerposition ist zu achten. Der Patient ist vor der Behandlung sorgfältig aufzuklären. Nach Durchführung der aus klinischchirurgischen Aspekten gebotenen Maßnahmen zur Vorbereitung des zu therapierenden Bereiches wird die Umverpackung geöffnet, ein Blister mit der lichtaktivierbaren Substanz, ein Blister mit einer Kanüle und gegebenenfalls der Beipackzettel entnommen. Dieser ist vor der Erstanwendung zu lesen. Unter Berücksichtigung steriler Kautelen sind beide Blister über dem sterilen OP-Tray zu entleeren. Spritze und Kanüle sind damit steril und zur Anwendung im sterilen Bereich geeignet. Der Silikonstopfen wird vorsichtig von der Spritze entnommen und die Kanüle durch Eindrehen auf dem Luerkonus fixiert.

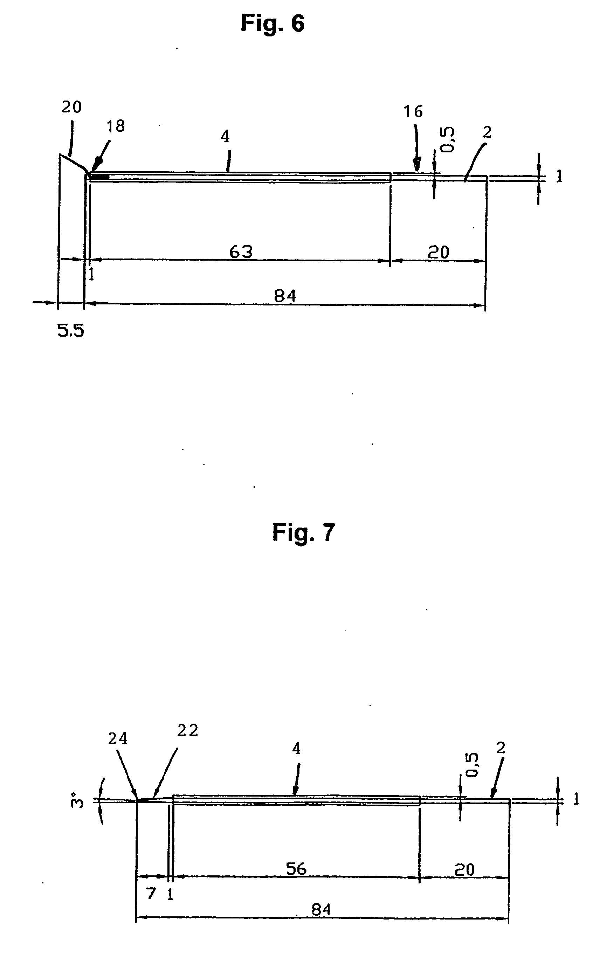

Fig. 6 zeigt das noch nicht gebogene Lichtleiterstück des Applikators ohne den Verbindungskörper, welcher in dem Bereich 16 angeordnet wird. Zwischen diesem Bereich 16 und dem freien Ende 18 ist der Schutzmantel 4 vorgesehen. Am freien Ende 18 ist ferner ein Abstandshalter 20 angeordnet, mittels welchem ein definierter Abstand, hier 5,5 mm, zu dem zu therapierenden Bereich vorgegeben wird.

Fig. 7 zeigt das Lichtleiterstück für die Pocketsonde, wobei das freie Ende 22 frei vorm Schutzmantel 4 ist und eine Länge von 7 mm aufweist. Das freie Ende 22 ist angeschliffen und weist eine Oberfläche entsprechend einer Bearbeitung mit Schleifpapier mit einer Korngröße 100 auf. Die Spitze 24 des freien Endes 22 ist stumpf ausgebildet und/oder mit einem Radius versehen. Die Oberfläche weist in bevorzugter Weise eine vorgegebene Mikrorauhigkeit auf. Diese besitzt vorzugsweise einen Ra-Wert im Bereich zwischen 10 bis 40 $\mu$m, vorzugsweise im Bereich zwischen

20 bis 30 $\mu$m.

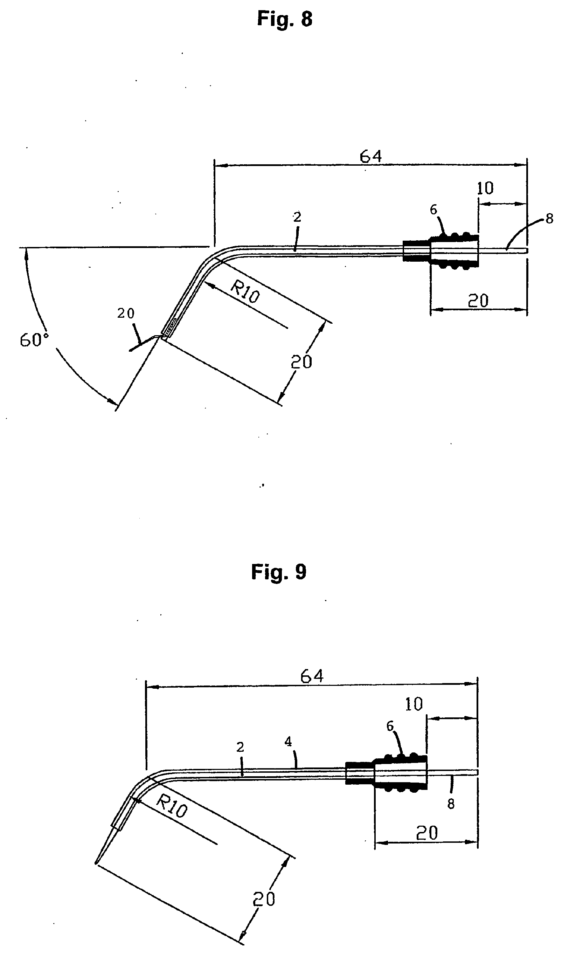

Fig. 8 zeigt einen Applikator ähnlich Fig. 4, wobei am freien Ende ein Abstandshalter 20 angeordnet ist. Das Lichtleiterende 8 ragt bei dieser Ausführungsform aus dem Verbindungskörper 6 heraus, und zwar hier in einer Länge von 10 mm.

In Fig. 9 ist ein gebogenes Lichtleiterstück einer Pocketsonde ähnlich Fig. 3 dargestellt, wobei das freie Lichtleiterende 8 aus dem Verbindungskörper 6 mit einer vorgegebenen Länge, hier 10 mm, herausragt.

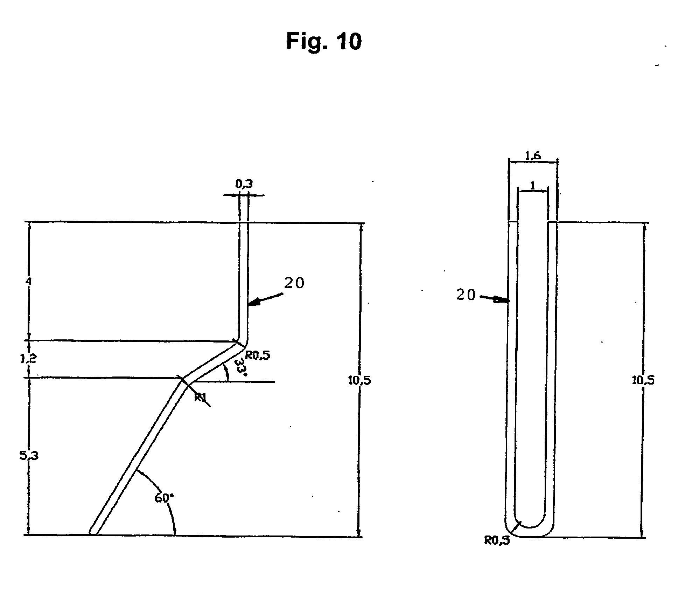

In Fig. 10 sind seitliche Ansichten einer bevorzugten Ausführungsform des Abstandshalters 20 dargestellt.

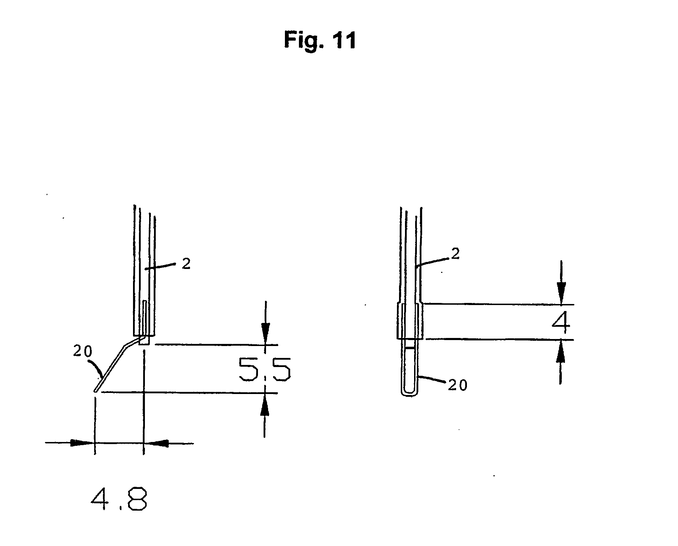

Fig. 11 zeigt teilweise in seitlichen Ansichten die Flächensonde mit eingeschrumpftem Lichtleiter mit dem Abstandshalter 20.

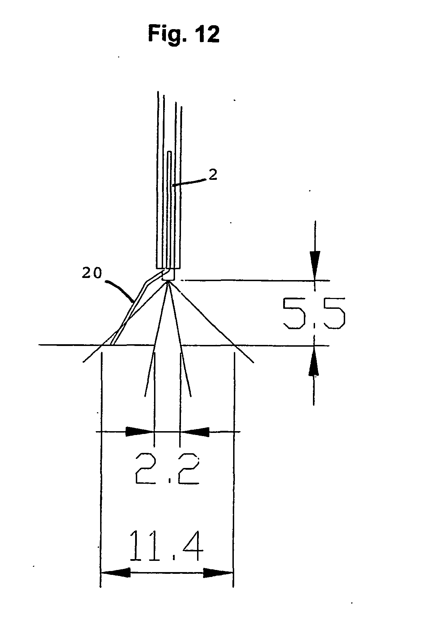

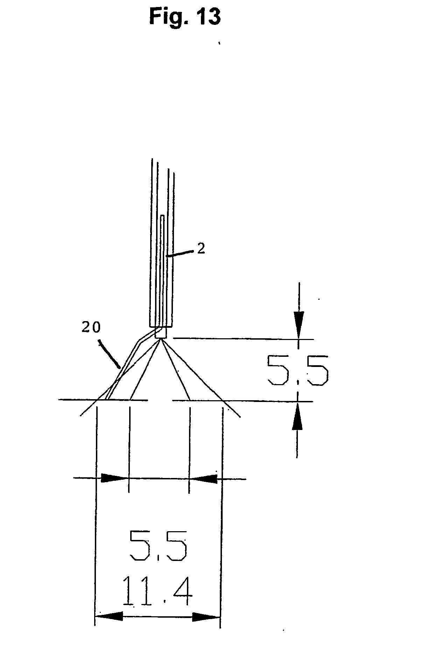

Fig. 12 und 13 zeigen teilweise Flächensonden, wobei gemäß Fig. 12 die Flächensonde eine numerische Apertur von 0,72 aufweist.

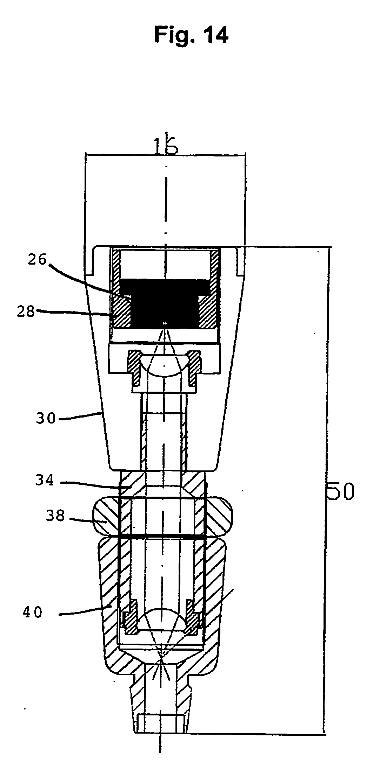

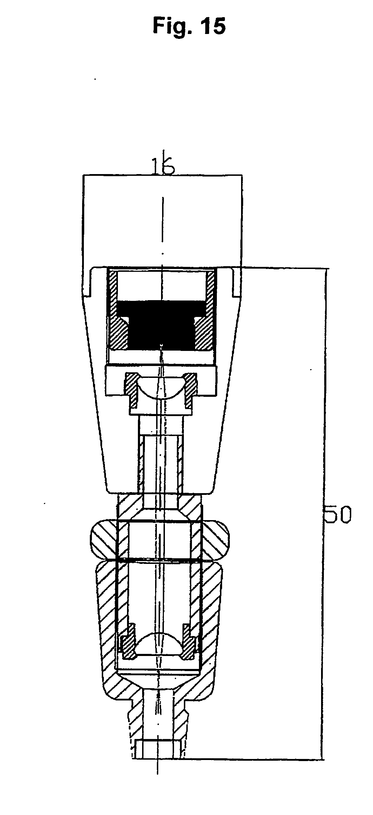

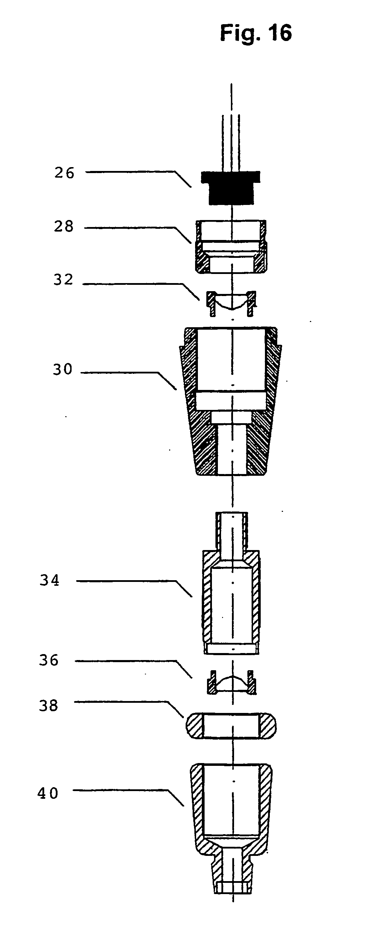

Das optische System des Bestrahlungsgeräts ist in Fig. 14 bis 16 dargestellt, wobei gemäß Fig. 14 eine Strahldivergenz senkrecht von maximal 35 und gemäß Fig. 15 eine Strahldivergenz parallel von maximal 10° vorgegeben ist. Wie insbesondere aus der Explosionszeichnung gemäß Fig. 16 ersichtlich, ist die Laserdiode 26 in einer Gewindehülse 28 angeordnet. Es handelt sich um eine Multimode Laserdiode von 100 mW kontinuierlich für 670 nm einschließlich einer Monitordiode. In einem Kegelteil 30 befindet sich eine Objektivlinse 32, wobei ferner ein Linsenhalter 34 für eine weitere Objektivlinse 36 vorgesehen ist. Des Weiteren sind ein Verstellring 38 sowie ein Aufnahmekörper 40 vorgesehen.

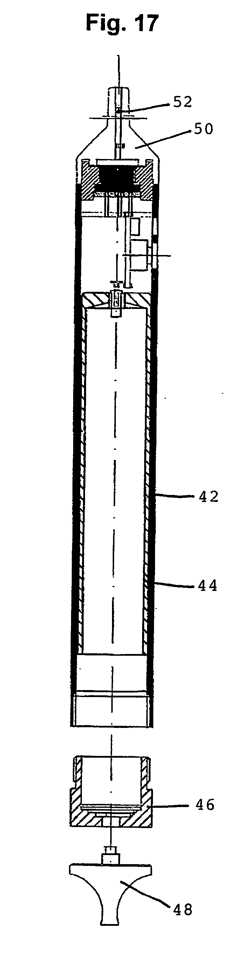

Fig. 17 zeigt das Bestrahlungsgerät ohne das optische System, wobei in einem Gehäuserohr 42 ein Batterierohr 44 für die zur Stromversorgung der Elektronik erforderlichen Batterien vorhanden ist. Am hinteren, gemäß Zeichnung unteren Ende des Gehäuserohres 42 ist eine Kappe 46 über eine Gewindeverbindung lösbar mit dem Gehäuserohr 42 verbunden bzw. verbindbar. Des Weiteren ist am hinteren Ende des Gehäuserohrs 42 bzw. der Kappe 46 ein Schlüsselschalter 48 vorgesehen, mittels welchem das Bestrahlungs- bzw. Lasergerät ein- oder ausschaltbar ist. Am vorderen Ende des Gehäuserohres, welches als Schutzgehäuse ausgebildet ist, ist ein Kopfteil 60 angeordnet, welches zur Aufnahme der Applikatoren und zur Lichtstrahlauskopplung durch die zentrale Bohrung 52 ausgebildet ist.

In Verbindung mit dem bevorzugt ausgebildeten Bestrahlungsgerät, welches auch als HEL-BOTherLite Laser bezeichnet wird, steht folgende Lichtleistung für die Belichtung zur Verfügung:

|  | Pocketsonde | Spotsonde |
|---|---|---|
| Abstrahlung | Radial, Bereich 250 - 280 Grad, insbesondere im wesentlichen 270 Grad frontal | Bereich 40-60 Grad, insbesondere im wesentlichen 50 Grad frontal |
| Leistungsdichte | >40mW/cm$^2$ | >40mW/cm$^2$ |

Die bevorzugt verwendete lichtaktivierbare Substanz ist eine sterile, isotonische, tiefblaue, geruchlose wässrige Flüssigkeit. Sie enthält Phenothiazin-5-ium, 3,7-bis (dimethylamino)-chlorid zur Einfärbung und Sensibilisierung von Mikroorganismen für die lethale photodynamische Therapie in Verbindung mit dem Bestrahlungsgerät.

1 ml der Lösung enthält:

- 1% Phenothiazin-5-ium, 3,7-bis (dimethylamino)-, chlorid
- Glucose zur Isotonisierung
- MHPC (Methylhydroxypropylcellulose) zur Einstellung der Viskosität
- Citrat zur Pufferung der Lösung.

Die Osmolarität entspricht ungefähr der von menschlichem Gewebe.

Die in Lösung mit der lichtaktivierbaren Substanz ist in einer Glasspritze verpackt und mit einem Stopfen aus Silikon verschlossen. Die Füllmenge beträgt 0,5 ml +/- 0,1 ml. Die Glasspritze ist einem Blister versiegelt und wird in einem validierten Sterilisationsprozess dampfsterilisiert. Die genannte Lösung ist zweckmäßig in fünf Blister mit einem Beipackzettel in einen Karton verpackt. 5 Kanülen 26G, die ebenfalls in je einem Blister verpackt und sterilisiert sind, sind beigepackt.

Die lichtaktivierbare Substanz dient im Rahmen der lethalen photodynamischen Lasertherapie zur Einfärbung und Sensibilisierung von Mikroorganismen bei lokalen oberflächlichen Infektionen, insbesondere im Mund-, Kiefer- und Gesichtsbereich. Durch die anschließende Belichtung mit dem Bestrahlungsgerät werden eingefärbte Mikroorganismen eliminiert und die natürliche Mundflora wiederhergestellt.

Die topische Applikation erfolgte im Bereich der Infektion ohne oder mit chirurgischer Eröffnung und Kürettage und paraläsional in folgender Weise: Der Patient spült mit Wasser zweimal für 20 Sekunden. An den zu behandelnden Flächen anhaftender Speichel oder Blut wird abgesaugt oder abgetupft, um eine Verdünnung von der photoaktiven Substanz zu verhindern.

Mit der Applikation der lichtaktivierbaren Substanz wird langsam begonnen, welche mittels Spritze flächendeckend auf die infizierten Gewebsareale aufgebracht wird. Die Menge ist unbedingt so zu wählen, dass der die lichtaktivierbare Substanz die Oberfläche der infizierten Bereiche möglichst dünn benetzt. Die vollständige Benetzung von Nischen und Taschen im Gewebe ist sicherzustellen. Gegebenenfalls bei komplexer Morphologie mit dem Luftbläser vorsichtig verteilen. Die Einwirkzeit der lichtaktivierbaren Substanz beträgt mindestens 60sec. Nach Spülung für mindestens 3 sec mit gleichzeitiger Absaugung überschüssiger Lösung (unbedingt Farbstoff-Depots entfernen!) mit dem Bestrahlungsgerät belichten. Wesentlich für die keimreduzierende Wirkung und damit für das Behandlungsergebnis, ist die richtige Dosierung der Energiezufuhr, die Belichtung.

Die Therapiekontrolle durch den Behandler enthält als wesentliche Größe für die Bestimmung der notwendigen Energiedichte (J/cm2) die Behandlungszeit entsprechend der vorgegebenen Behandlungsfläche. Mindestens 1 Minute Belichtungszeit mit dem Bestrahlungsgerät pro $cm^2$ oder pro Zahn sind einzuhalten.

Für die Berechnung der bestrahlten Fläche A einer Läsion mit einem Radius r während der Bestrahlung werden folgende Formeln eingesetzt :

a.: mit der Pocketsonde: Fläche des Defektes F = Breite B * Höhe*2 und damit pro Quadrant :

| [Zähne | 1 | 2 | 3 | 4 | 5 | 6 | 7 | 8 |
|---|---|---|---|---|---|---|---|---|
| Breite cm | 0,6 | 1,2 | 1,8 | 2,4 | 3 | 3,6 | 4,2 | 4,8 |
| Höhe cm | 0,8 | 0,8 | 0.8 | 0,8 | 0,8 | 0,8 | 0.8 | 0,8 |
| 3DFaktor | 2 | 2 | 2 | 2 | 2 | 2 | 2 | 2 |
| Fläche $cm^2$ | 0,96 | 1,92 | 2,88 | 3,84 | 4,8 | 5,76 | 6,72 | 7,68 |
| Leistung mW | 50 | 50 | 50 | 50 | 50 | 50 | 50 | 50 |
| Flächendichte W/cm$^2$ | 0,052 | 0,052 | 0,052 | 0,052 | 0,052 | 0,052 | 0,052 | 0,052 |
| Belichtung sec | 60 | 120 | 180 | 240 | 300 | 360 | 420 | 480 |
| Energiedichte J/cm$^2$ | 3,125 | 3,125 | 3,13 | 3,125 | 3,125 | 3,125 | 3,125 | 3,125 |

Belichtungsprotokoll für die Pocketsonde

b.: mit der Spotsonde: Fläche des Defektes F = ($\pi r^2$)

Die Leistungsdichte (FD) errechnet sich nach:

$$FD\ (Watt/cm^2) = Leistung\ (Watt)/\ bestrahlte\ Fläche\ (cm2)$$

Die Energiedichte (ED) errechnet sich nach:

$$ED\ (Wattsec/cm^2) = Leistung\ (Watt)*Zeit\ sec/\ bestrahlte\ Fläche\ (cm^2)$$

Die jeweils gewählte Dosimetrie ist in der folgenden Tabelle zusammengestellt. In der Regel wird in einem Abstand von 0,55 cm bestrahlt und bei einer Leistungsdichte von 0,051 W/cm2, eine Gesamtdosis von 3 J/cm2 verwendet.

| Bestrahlungseinheit | 1 | 1 | 1 | 2 | 3 | 4 | 5 | 6 |
|---|---|---|---|---|---|---|---|---|
| Durchmesser cm | 1,8 | 0,9 | 1 | 1 | 1 | 1 | 1 | 1 |
| Radius cm | 0,9 | 0,45 | 0,5 | 0,5 | 0.5 | 0,5 | 0,5 | 0,5 |
| Abstand cm | 1 | 0,5 | 0,55 | 0,55 | 0,55 | 0,55 | 0,55 | 0,55 |
| Fläche $cm^2$ | 2,5434 | 0,6359 | 0,79 | 1,57 | 2,355 | 3,14 | 3,925 | 4,71 |
| Leistung mW | 40 | 40 | 40 | 40 | 40 | 40 | 40 | 40 |
| Flächendichte W/cm$^2$ | 0.016 | 0,063 | 0,051 | 0,051 | 0,051 | 0,051 | 0,051 | 0,051 |
| Belichtung sec | 60 | 60 | 60 | 120 | 180 | 240 | 300 | 360 |
| Energiedichte J/cm$^2$ | 0,94 | 3,77 | 3,06 | 3,06 | 3,06 | 3,06 | 3,06 | 3,06 |

Belichtungsprotokoll für die Spotsonde

Werden Bakterien durch lichtaktivierbare Substanzen bzw. Vitalfarbstoffe wie MB angefärbt und mit Licht geeigneter Wellenlänge bestrahlt, kann eine effektive phototoxische Wirkung induziert werden. Ungefärbten Zellen weisen keine toxischen Schädigungen auf. Von der photochemischen Abtötung möglicherweise pathogener Bakterien macht man in Pelztierfarmen oder Zoologischen Gärten Gebrauch, indem das Trinkwasser mit Methylenblau versetzt wird.

Als Photosensibilisator zur lokalen Behandlung von herpesbedingten Erkrankungen wird MB seit 25 Jahren eingesetzt. Die Dunkeltoxizität und Phototoxizität (PDT) von intratumoral appliziertem Methylenblau wurde experimentell an Kolontumoren untersucht. Eine Zerstörung dieser Tumoren nur durch Bestrahlung mit geringer Gesamtdosis (6 J/cm2) oder durch alleinige Verabreichung der Substanz (20 µg/ml) wurde nicht erreicht.

Die Gabe von MB kann zu systemischen Nebenwirkungen wie erhöhter Schweißbildung, Übelkeit und Erbrechen führen.

Bei oraler Verabreichung kann es zu gastrointestinalen Beschwerden und zu Dysurie kommen. In der Zubereitung der Lösung vorhandene Inhaltsstoffe sind:

| Wirk- und Hilfsstoffe | 1ml enthält [mg] |
|---|---|
| Methylenblau x 3 H2O | 10,00 |
| Trinatriumcitrat x 2 H2O | 0,433 |
| Citronensäure x 1H2O | 1,667 |
| MHPC | 10,00 |
| Natriumchlorid | 9,00 |
| Wasser für Injektionszwecke | 1000 |

Inhaltsstoffe der lichtaktivierbaren Substanz:

Nach der vorliegenden Biokompatibilitätsbewertung sind die in der Zubereitung verwendeten Substanzen hinsichtlich ihrer Biokompatibilität, Teratogenität und Mutagenität unter den gegebenen Anwendungsbedingungen als akzeptabel hinsichtlich des erstrebten Behandlungsziels beurteilt.

Mit der topischen Applikation des Photosensibilisators (PS) entfallen wesentliche Probleme der systemischen Anwendung eines Medikaments wie von Antibiotika, aber auch von systemisch eingesetzten PS, wie z. B Substanztoxizität und generalisierte, mehrwöchige Photosensibilisierung der Haut. Durch die topische Applikation lässt sich die Spezifität erhöhen, so dass gesundes Gewebe, z. B Schleimhaut in der Umgebung der Läsion, geschont wird.

Aufgrund der geringen Nebenwirkungen kann wiederholt behandelt werden. Die Therapie zeichnet sich weiterhin durch Nicht-Invasivität aus.

Die lokale Konzentration wird auch dadurch beeinflusst, dass nach lokaler Applikation des PS in der Regel eine erhöhte Speichelbildung der Mundschleimhaut induziert wird. Diese führt zur Verringerung der PS-Konzentration und vermindert die Penetration des Farbstoffs in die Läsion. Darüber hinaus können Speichelproteine durch unspezifische Bindung eine Inaktivierung des PS hervorrufen. Durch die Einbringung des PS in eine, insbesondere viskose Lösung wird erfindungsgemäß die Vermischung, Verdünnung und Reaktion mit Speichel für die Behandlungszeit verringert.

Nach der Einwirkzeit, die mit mindestens 60 sec angegeben ist, wird erfindungsgemäß der überschüssige PS entfernt, um die Lichttransparenz des behandelten Gewebes zu erhöhen.

Messungen zeigen, dass ein auf dem Gewebe stehender Flüssigkeitsfilm der Lösung mit der lichtaktivierenden Substanz von 100 µm die effektive Energiedichte um 97% verringert. Bei weiterer Verdopplung der Schichtdicke wird jeweils, gemäß dem Lambert-Beerschen Gesetz, das Licht weiter geschwächt. Somit ist eine therapeutisch wirksame Bestrahlung bei überschüssiger Lösung mit der lichtaktivierenden Substanz nicht möglich.

Für die effektive PDT-Applikation von Mundschleimhautläsionen wird eine Energiedichte von 50-100 J/cm2 empfohlen. Hierbei ist die Energiedosis der Art und der Lokalisation des Befundes anzupassen. Da die Mundschleimhaut generell sehr schmerzempfindlich ist, sollten Leistungsdichten oberhalb 150 mW/cm$^2$ vermieden werden. Leistungsdichten zwischen 200 und 500 mW/cm$^2$ können zu unspezifischen thermischen Gewebeschäden führen.

Um eine gleichmäßige Dosis im Bereich der Läsion zu erreichen, soll die Bestrahlungsfläche jeweils größer als die Fläche der Läsion gewählt werden.

Die Lichtdosis von ca. 100 J/cm2 für die PDT lässt sich auf unterschiedliche Weise erzielen: Hohe Leistungsdichte und kurze Expositionszeiten oder niedrige Leistungsdichte und lange Expositionszeiten. Hohe Leistungen scheiden wegen der erwähnten thermischen Schäden aus. Andererseits lässt sich bei zu niedrigen Leistungsdichten ein photodynamischer Effekt auch bei entsprechend langen Bestrahlungszeiten nicht erzielen.

Methylenblaulösungen sind in der Lage, die Zahl aller untersuchten Mikrooganismen in den jeweiligen Kulturmedien zu reduzieren.

Methylenblau -Lösungen reduzieren in vitro nahezu alle grampositiven Bakterien bei einer Konzentration von 25-44 μmol. Die vollständige Reduzierung gramnegativer Keime verlangt 3- 30 fach höhere Konzentrationen. P.aeruginosa wurde bei einer Konzentration von 200 ~mol und einer Energiedichte von 100 mW/cm$^2$ um 3,5 log 10 CFU reduziert.

Die beobachtete Dunkeltoxizität war für Toluidinblau (TB) größer als für Methylenblau (MB). Dies stimmte mit dem Verteilungskoeffizient P überein, der für TB mit 0,33 und für MB mit 0,11 bestimmt worden war.

Da log P< 0 war sind somit beide Farbstoffe als hydrophil zu bezeichnen, und sollten, zumindest theoretisch die wassergefüllten Porin-Proteinkanäle gramnegativer Bakterien passieren können.

Während die Dunkeltoxizität für grampositive Bakterien kaum vom Typ abhängig war, war die Dunkeltoxizität für gramnegative Bakterien ganz deutlich vom Typ abhängig und zwar entsprechend dem transmembranen Permeabilitätskoeffizienten der äußeren Membran gramnegativer Bakterien.

Die Dunkeltoxizität war sowohl von der Konzentration als auch der Inkubationszeit vor Bestrahlung abhängig.

S.aureus wurde für die grampositive Gruppe als das resistentestes Bakterium identifiziert, dessen Vernichtung die höchsten Konzentrationen verlangte.

P .aeruginosa wurde für die gramnegative Gruppe als das resistentestes Bakterium identifiziert, dessen Vernichtung die höchsten Konzentrationen verlangte.

Bei gramnegativen Bakterien hängt die photodynamische Sensitivität von der transmembranen Permeabilität ab und die Hydrophilizität, die positive Ladung und das niedrige Molekulargewicht der Farbstoffmoleküle begünstigt die Wirksamkeit.

Für den vorliegenden Therapieansatz werden für die Behandlung mikrobiell infizierter Bereiche nur Einwirkungszeiten von 60 sec. mit anschließender Spülung vor der Belichtung vorgesehen.

Die gewählten Parameter der Therapie, insbesondere

- Konzentration der lichtaktivierenden Substanz in Lösung von 1 %
- eine Energie von 2,4 J,
- einer Leistungsdichte von 50mW/cm$^2$
- und einer Energiedichte von 3 J/cm$^2$
- die Inkubationszeit von 60 sec mit anschließenden Ausspülen der Lösung

sind geeignet zur Gewährleistung eines sicheren Behandlungsergebnisses. Andererseits sind bei Einhaltung dieser Randbedingungen mögliche Risiken und Nebenwirkungen begrenzt und scheinen im Lichte der zu erwarteten positiven Aspekte für den Patienten nach entsprechender Aufklärung hinnehmbar.

Die PDT basiert auf einem photochemischen Prozess, bei dem Photosensibilisatoren (PS) mittels Laserstrahlung aktiviert werden und die eingestrahlte Energie so "portionieren", dass sie für die Bildung lokal toxisch wirkender Sauerstoffradikale zur Verfügung steht. Bei den gewählten Energie -und Leistungsdichten von 3 J/cm$^2$ bzw 50mW/cm$^2$ sind thermische Schädigungen des Gewebes sicher aufzuschließen.

Die klinische Anwendung der PDT ist möglich, da die Farbstofflösungen Zellsysteme selektiv einfärben, andererseits die Wechselwirkung mit Epithel sehr begrenzt ist. Untersuchungen an normaler Mundschleimhaut zeigten dass die Eindringtiefe von MB -Lösungen nach 10 min Inkubationszeit nur auf die ersten 1-2 äußeren Zeillagen des Epithels begrenzt war. Weiterhin ist die Lebensdauer der aktiven Radikale und ihrer Vorstufen unter Mikrosekunden, so dass auch eine Ausdehnung der destruktiven Effekte ins gesunde Gewebe durch Diffusion praktisch ausgeschlossen ist, da die nötige Zeit nicht zur Verfügung steht.

Die Wirkung ist somit an die Anwesenheit der Farbstoffmoleküle des PS gebunden.

Die Wahl von beispielsweise Methylenblau als photodynamisch aktiver Substanz der Zubereitung begründet sich einerseits in der geringen Toxizität von Methylenblau unter den gewählten Behandlungsbedingungen, anderseits in der günstigen Lage des Absorptionsmaximums bei 664 nm:

- es stehen für diese Wellenlänge leistungsfähige und effiziente Dioden zur Erzeugung des Laserstrahles zur Verfügung

- es wird im Bereich des sichtbaren Lichtes behandelt, was entscheidend zur Sicherheit und Effizienz der Therapie beiträgt

- die Eindringtiefe des Lichtes ins Gewebe ist in diesem Wellenlängenbereich ausreichend tief um auch penetrierende Keimbesiedelung erreichen zu können

- die Singulettsauerstoffbildung ist der wesentliche Wirkmechanismus der Keimtötung wobei andererseits gesunde Zellen diese Radikale durch Katalasen abbauen

- können, andererseits auch durch die Wirksamkeit von Vitaminen wie Vitamin C und E ein gewisser Schutz besteht.

- anhand der Ergebnisse der spektralphotometrischen Untersuchungen MB mit und ohne Bestrahlungen erfolgte eine annähernd lineare Abnahme der Extinktion mit Zunahme der applizierten Energiedichte. Photobleaching mit Destruktion der Farbstoffmolekühle findet im wesentlichen Umfang bei Energiedichten statt, die um einen Faktor 7 über der therapeutisch aufgebrachten Energiedichte liegt.

- findet in diesem Bereich ein unterstützender photobiologischer Effekt statt, der die Gewebsregeneration fördert und die lokale Stoffwechsellage stabilisiert

- Methylenblau steht in reiner und dokumentierter Form zur Verfügung .die Verwendung in einer Zubereitung führt zu stabilen Lösungen

- diese Lösungen sind unter den Bedingungen des ärztlichen Umfeldes bei Anwendung angezeigter Sorgfalt einfach und sicher zu handhaben

- die bei der Anwendung anfallenden Abfälle sind relativ unschädlich

Durch die Abstimmung von Farbstoff und Lichtquelle steht ein therapeutisch effektives System zur Verfügung, welches sowohl gegen gram positive und gram negative Bakterien, als auch gegen Pilze wie candida albicans Wirkung zeigt und in der Lage ist, durch die Reduktion der Mikroorganismen die körpereigene Abwehr zeitlich begrenzt zu unterstützen um die klinische Symptomatik zu verbessern.

Mit den Applikatoren HELBO*PocketProbe* ist es möglich, erfindungsgemäß eine im wesentlichen im Bereich von 1 bis 7 J/cm$^2$, vorzugsweise von 2 bis 4 J/m$^2$, insbesondere im wesentlichen eine gleichmäßige Energiedichte von 3 J/cm$^2$ auch in den komplexen Arealen um und zwischen Zähnen im hinteren Bereichen der Mundhöhle zu applizieren.

Durch den Einsatz der photodynamischen Therapie können durch unterstützende photobiologische Effekte rasche Schmerzfreiheit und eine beschleunigte Wundheilung beobachtet werden.

Als Nebenwirkungen während der Therapie wurde gelegentliches Brennen beobachtet, das jedoch nach Ende der Behandlung rasch abklingt.

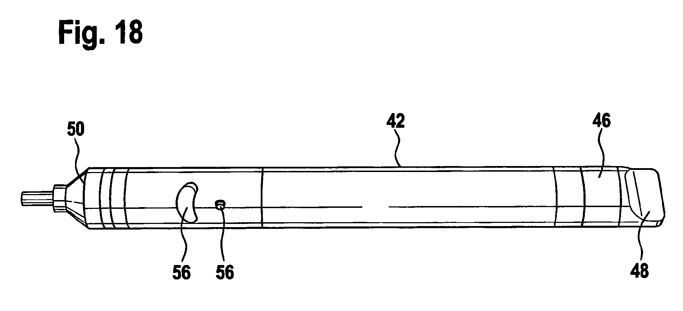

Eine besondere Ausgestaltung der Erfindung wird nachfolgend anhand der Fig. 18 bis 27 näher erläutert. Das Bestrahlungsgerät, welches nachfolgend auch als Therapielaser bezeichnet wird, dient zur photodynamischen Therapie (PDT) und ist insbesondere als Lasergerät ausgebildet. Es enthält eine Sperreinrichtung, mittels welcher bei Ankoppeln eines Applikators, welcher einen Lichtleiter enthält, selbsttätig der Lichtweg freigegeben wird. Solange der Applikator und/oder der Lichtleiter an das Bestrahlungsgerät bzw. Lasergerät nicht angekoppelt ist, ist mittels der erfindungsgemäß ausgebildeten Sperreinrichtung der Austritt von Laserlicht aus dem Lasergerät blockiert. Die Sperreinrichtung enthält insbesondere einen Verriegelungskörper, welcher derart ausgebildet und angeordnet ist, dass nur bei angekoppeltem Applikator bzw. Lichtleiter Laserlicht austreten kann. Der Verriegelungskörper der Sperreinrichtung ist in einer labilen Position angeordnet, aus welcher er ausschließlich bei ordnungsmäßer Ankopplung des Applikators bzw. Lichtleiters zur Freigabe des Lichtweges bringbar ist. Die Sperreinrichtung ist insbesondere in das Kopfteil des Bestrahlungsgeräts integriert. Alternativ kann die Sperreinrichtung an anderer Stelle des Bestrahlungsgeräts und/oder anders ausgebildet sein, und insbesondere in das optische System integriert sein. Das erfindungsgemäße Bestrahlungsgerät gewährleistet eine direkte Einkopplung des Lichtes aus der Diode bzw. Laserdiode in den Lichtleiter derart, dass

1. geringe Verluste dadurch entstehen, dass eine Lichtleitfaser hoher numerischer Apertur zum Einsatz gelangt, insbesondere größer als 0,5, bevorzugt größer als 0,7, wodurch gewährleistet ist, dass eine relativ große Fläche gleichmäßig bestrahlt wird, da der Lichtstrahl bei Austritt aus der Faser stark öffnet,

2. nur bei Einbringung bzw. Ankopplung des Lichtleiters wird mittels der vorgebauten Sperreinrichtung der Lichtaustritt ermöglicht, und ansonsten wird ein direkter Lichtaustritt aus dem Lasergerät unterbunden. Somit kann das Lasergerät ohne Schutzbrille betrieben werden und ohne dass ein Laserschutzbeauftragter zu benennen ist, wie es bei der vorgesehenen Leistung des Lasergerätes grundsätzlich nötig wäre.

Aufgrund der hohen numerischen Apertur des zum Einsatz gelangenden Lichtleiters wird zum einen die erwähnte Streuwirkung beim Lichtaustritt bzw. Beleuchtung auf dem zu therapierenden Bereich erreicht und zum anderen auf der dem Lasergerät zugewandten Seite des Applikators bzw. des Lichtleiters eine Sammelwirkung derart sichergestellt, dass ein Linsensystem zwischen dem Laser bzw. der Diode und dem Lichtleiter entbehrlich ist.

Beim Einsatz des Bestrahlungs- bzw. Lasergerätes und/oder der Verwendung der Anordnung zur photodynamischen Therapie sind die nachfolgend aufgeführten Schritte von besonderer Bedeutung:

1. Die Lösung mit dem Photosensitizer wird zunächst in hoher Konzentration auf den zu therapierenden Bereich aufgebracht, damit jene möglichst schnell eindringt, insbesondere in den Plaque von Zähnen.

2. Zusätzlich wird bevorzugt eine Spülung mit einem Medium, insbesondere Wasser, möglichst geringer Ionenkonzentration durchgeführt, damit Bakterien und/oder Zellmembranen aufgrund des somit erzeugten osmotischen Druck-Gradienten gefährdet werden. Es sei festgehalten, dass beispielsweise eine physiologische Kochsalzlösung aufgrund der relativ hohen Ionenkonzentration ungünstig wäre. Der pH-Wert des Mediums wird vorteilhaft eher basisch vorgegeben. Bevorzugt wird ein pH-Wert von 7 bis 9 vorgegeben. Der Sauerstoffpartialdruck ist bevorzugt groß vorgegeben. Im Rahmen der Erfindung weist das Medium, insbesondere aufbereitetes Leitungswasser, zur Spülung einen Sauerstoffpartialdruck im Bereich von 4 bis 6 mg/l auf. Zweckmäßig ist das Medium angereichert mit molekularem Sauerstoff bis zu 14 mg/l. Weiterhin hat sich erfindungsgemäß eine Peroxidanreicherung als zweckmäßig erwiesen, und zwar bevorzugt als 0,5% bis 3%ige Wasserstoffperoxidlösung.

3. Aufgrund der vorherigen Spülung mit einem Medium geringer Konzentration wird bei der Bestrahlung mittels des Laserlichts eine optimierte Zellschädigung durchgeführt.

Beschreibung des Bestrahlungs- bzw. Lasergerätes

Das Gerät wird mit Batterien bzw. Akkumulatoren betrieben und verwendet als Licht- oder Strahlquelle einen Halbleiterlaser (Laserdiode), der kontinuierlich betrieben wird (cw). Fig. 18 zeigt das Bestrahlungs- bzw. Lasergerät von der Seite ohne Applikator. Die Strahlquelle ist in einem zylindrischen, metallenen Schutzgehäuse 42 eingebaut. Die Länge des das Gehäuserohr 42 enthaltenden Schutzgehäuses beträgt etwa 124 mm, der Durchmesser etwa 16 mm. Am Ende des Schutzgehäuses 42, wo die Batterien bzw. Akkumulatoren einzulegen sind, wird eine Kontaktkappe 46 nach dem Einlegen der Batterien aufgeschraubt. Der betriebsbereite Zustand wird durch das Einsetzen des als Abschlusskappe geformten Schlüsselschalters 48 in die Kontaktkappe 46 erreicht. Der Betriebszustand wird durch verschiedenfarbige LEDs 54 angezeigt.

Am anderen Ende des Schutzgehäuses 42 ist das Kopfteil 50 auf das Schutzgehäuse 42 geschraubt und mit diesem verklebt, sodass der direkte Zugang zur Strahlquelle verhindert wird. Das Kopfteil 50 dient einerseits zur Aufnahme der Applikatoren und damit der Laserstrahlauskopplung, andererseits beinhaltet er den Verriegelungsmechanismus. Durch Drücken eines Tasters 56 wird schließlich der Halbleiterlaser aktiviert.

Applikatoren

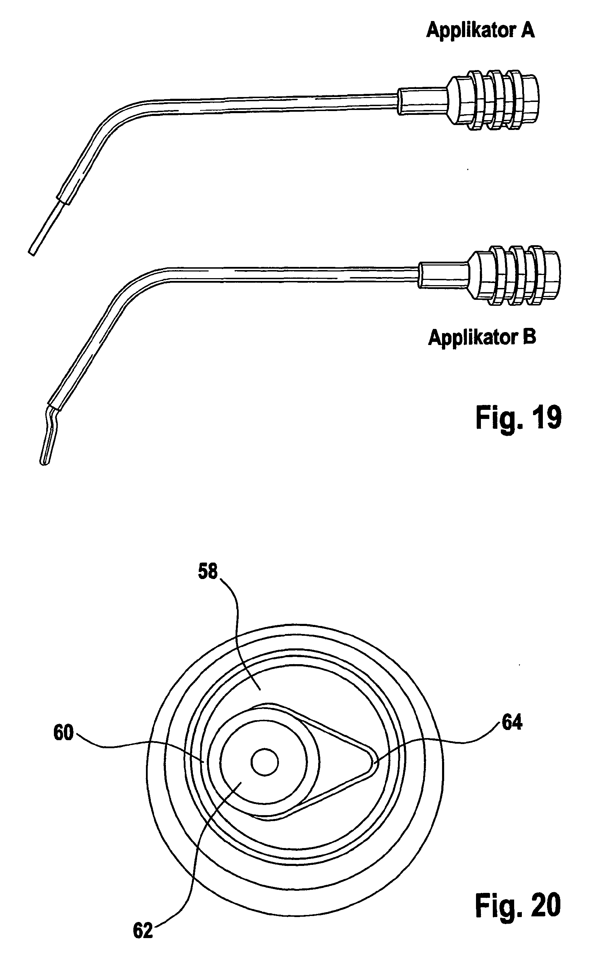

Fig. 19 zeigt zwei Ausführungsbeispiele von Applikatoren, wobei der im oberen Teil abgebildete Applikator A eine Pocketsonde ähnlich Fig. 2 ist, während der im unteren Teil abgebildete Applikator B eine Flächensonde ähnlich Fig. 3 ist. Beide Applikatoren bestehen aus einem transparenten Kunststofflichtleiter mit einem Durchmesser von etwa 1 mm, haben einen Luersteckerzur Aufnahme am Kopf des Lasergerätes, sind gebogen und mit einem weißen Schutzmantel zwischen Luerstecker und Strahlaustrittsende umgeben. Das Lichtleiterende (ohne Schutzmantel) beim Luerstecker wird in den Kopf des Schutzgehäuses gesteckt.

Applikator A weist eine leicht konische Spitze auf, deren Oberfläche auf den letzten 5 mm aufgeraut ist. Die raue Oberfläche bewirkt, dass das Laserlicht in nahezu alle Raumrichtungen abgestrahlt wird, wobei in axiale Richtung des Lichtleiters die meiste Energie abgegeben wird. Applikator B hat eine ebene Stirnfläche als Austrittsfläche für das Laserlicht. Zusätzlich ist eine Drahtschleife am Lichtleiterende als Abstandshalter eingebaut. Im Gegensatz zu Applikator A weist das Laserlicht eine divergierende, konische Abstrahlcharakteristik auf, wodurch in axiale Richtung mehr Energie abgegeben wird. Für alle weiteren Messungen wurde daher Applikator B verwendet, da er aus Sicht der Lasersicherheit das größere Gefahrenpotential beinhaltet.

Sperreinrichtung / Verriegelungsmechanismus

Der in Fig. 20 dargestellte Verriegelungsmechanismus enthält einen im Querschnitt "H"-förmigen, rotationssymmetrischen Verriegelungskörper 58, der in der Mitte ein Loch 60 mit 1,01 +0,02 mm Durchmesser hat. In der Ausnehmung des "H's", das dem Strahlaustritt abgewandt ist, ist die Laserdiode positioniert und emittiert Licht im aktivierten Zustand durch das Loch 60 im Verriegelungskörper 58 in Richtung Strahlaustritt.

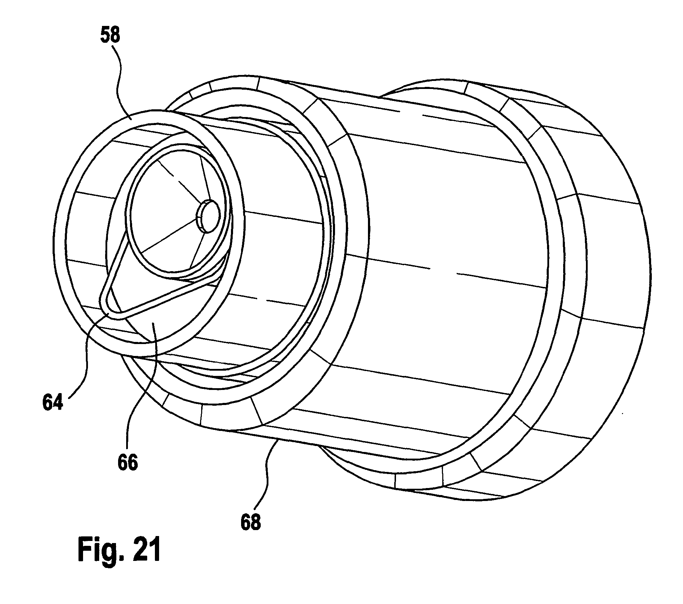

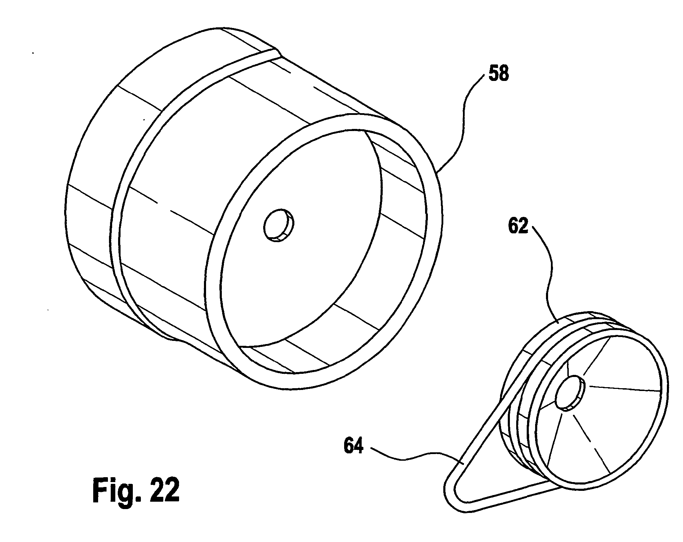

In der Vertiefung des "H's", die dem Strahlaustritt zugewandt ist, befindet sich eine runde Verriegelungsscheibe 62, ebenfalls mit einem in der Mitte befindlichen Loch mit 1 ,01 +0,02 mm Durchmesser. Der Außendurchmesser dieser Scheibe 62 ist wesentlich kleiner als der Innendurchmesser des Verriegelungskörpers 58, sodass gemäß Fig. 20 sich die Scheibe in der Vertiefung verschieben läßt. Die Verriegelungsscheibe wird jedoch mittels einer Drahtfeder (Verriegelungsfeder) azentrisch gehalten. Damit deckt diese Scheibe 62 das Loch des Verriegelungskörpers ab und das Laserlicht kann nicht austreten. Die Drahtfeder 64 wird in einer Nut am Umfang der Verriegelungsscheibe geführt. Wie aus Fig. 21 und der explosionsartigen Darstellung gemäß Fig. 22 des Verriegelungskörpers 58 und der Verriegelungsscheibe 62 ersichtlich, ist der Verriegelungskörper 58, in dessen Ausnehmung die Verriegelungsschreibe bewegbar angeordnet ist, in einem Diodenhalter 68 angeordnet. In der explosionsartigen Darstellung der Fig. 22 sind die beiden genannten Löcher des Verriegelungskörpers 58 und der Verriegelungsscheibe 62 gut zu erkennen.

Erst durch die Einführung des Lichtleiters des Applikators bis zum Anschlag wird die Verriegelungsscheibe 62 in die zentrale Position gedrückt, so dass sich das Loch der Verriegelungsscheibe 62 und das Loch des Verriegelungskörpers 58 überdecken und das Laserlicht in den Lichtleiter eingekoppelt werden kann. Wird der Lichtleiter herausgezogen, dann drückt die Drahtfeder 64 die Verriegelungsscheibe 62 in die Ausgangsposition zurück, und das Laserlicht wird abgeblockt. Anstelle der hier dargestellten Drahtfeder 64 können im Rahmen der Erfindung auch andere Rückführelemente vorgesehen sein, um den Austritt von Laserlicht nur dann zu ermöglichen, wenn der Applikator und insbesondere dessen Lichtleiterende ordnungsgemäß mit dem Bestrahlungsgerät verbunden ist.

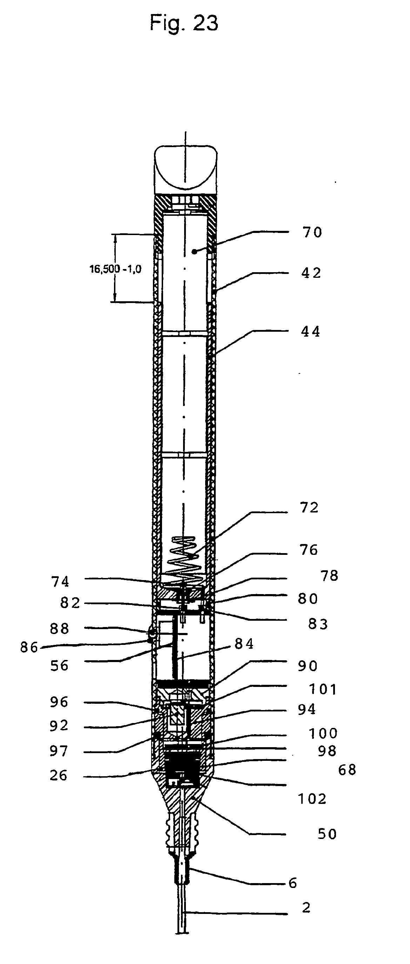

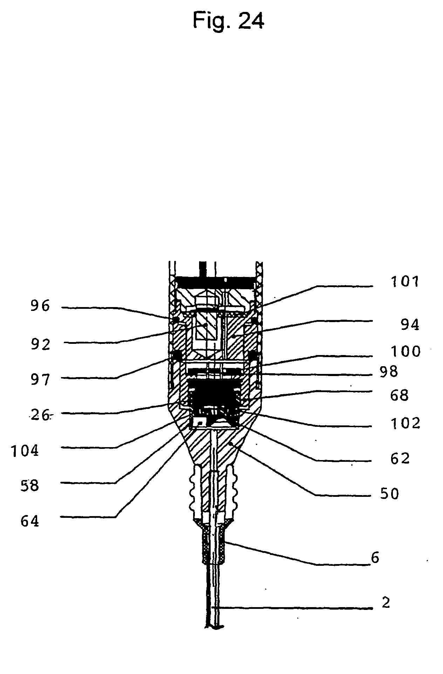

Fig. 23 zeigt einen Schnitt durch das Bestrahlungsgerät und Fig. 24 zeigt vergrößert dessen Vorderteil. Dieses Bestrahlungsgerät entspricht grundsätzlich dem oben bereits Erläuterten und enthält zusätzlich die Sperreinrichtung bzw. den Verriegelungsmechanismus. In dem vom Gehäuserohr 42 umgebenen Batterierohr 44 sind drei Batterien 70 angeordnet, welche mittels einer Batteriefeder 72 beaufschlagt sind. Zur Kontaktierung ist eine Steckerbuchse 74 in einer Buchsenführung 76 angeordnet, wobei ferner eine Scheibe 78, eine Distanzscheibe 80 vorhanden sind. Des Weiteren sind zwei Steckerstifte 82, 83 zur Kontaktierung mit einer Elektronik oder Elektronikplatine 84 vorgesehen. Auf der Elektronikplatine 84 ist der von außen betätigbare Taster 56 angeordnet, wobei insbesondere zur Abdichtung einer Tasterfolie 86 zusammen mit einer Kugel 88 vorgesehen sind. Nach vorn anschließend an den Innenbereich mit der Elektronik 84 und abgetrennt mittels einer hinteren Isolierung 90 ist ein Bereich mit einem Schlagbolzen 92 vorgesehen, wobei ferner eine vordere Isolierung 94 vorhanden ist. Innerhalb des Gehäuserohres 42 sind ferner zwei O-Ringe 96, 97 angeordnet. Am vorderen Ende des Gehäuserohres 42 ist das Kopfteil 60 angeordnet, dessen vorderes Ende in den Verbindungskörper bzw. Luerstecker 4 des hier nicht weiter dargestellten Applikators eingreift. Im Kopfteil 50 ist der bereits erläuterte Diodenhalter 68 mit der Laserdiode 26 angeordnet, wobei nach hinten zum Batterierohr 44 hin eine Isolierscheibe 98 sowie ein Print 100 für die Diode einschließlich zur Kontaktierung erforderlicher Drähte vorgesehen ist. Zusätzlich ist in Strahlungsrichtung vor der Laserdiode eine Schutzfolie 102 vorgesehen, mittels welcher in bevorzugter Weise die Laserdiode gegen äußere Einwirkungen geschützt wird, wobei zusätzlich ein O-Ring 104 vorgesehen ist. Des Weiteren sind in der inneren Ausnehmung des Kopfteils 50 der bereits erläuterte Verriegelungskörper 58, die Verriegelungsscheibe 62 sowie die Verriegelungsfeder 64 angeordnet.

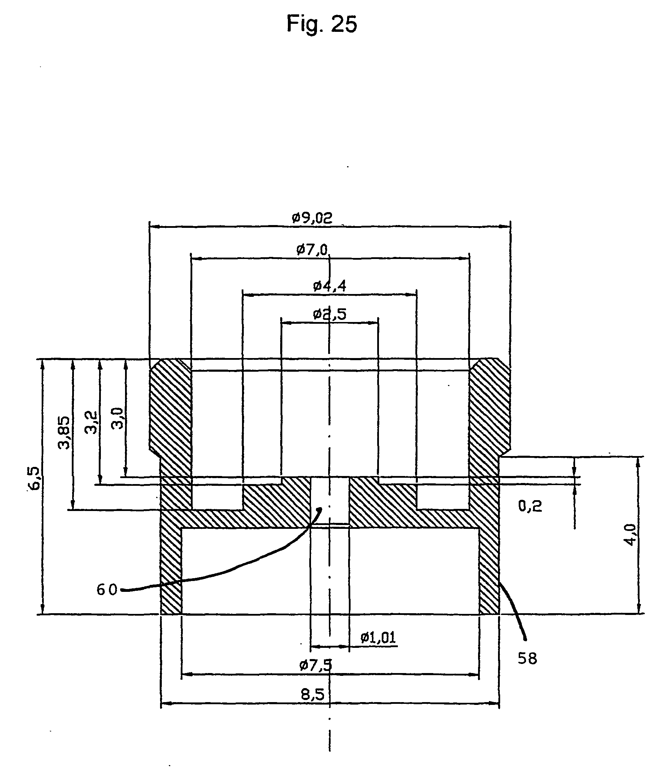

Fig. 25 zeigt einen Schnitt in einer axialen Schnittebene durch den Verriegelungskörper 58, wobei dessen H-förmige Querschnittsfläche gut zu erkennen ist. Es versteht sich, dass die für die besondere Ausgestaltung angegebenen Abmessungen in Millimeter auch abweichend vorgegeben werden können.

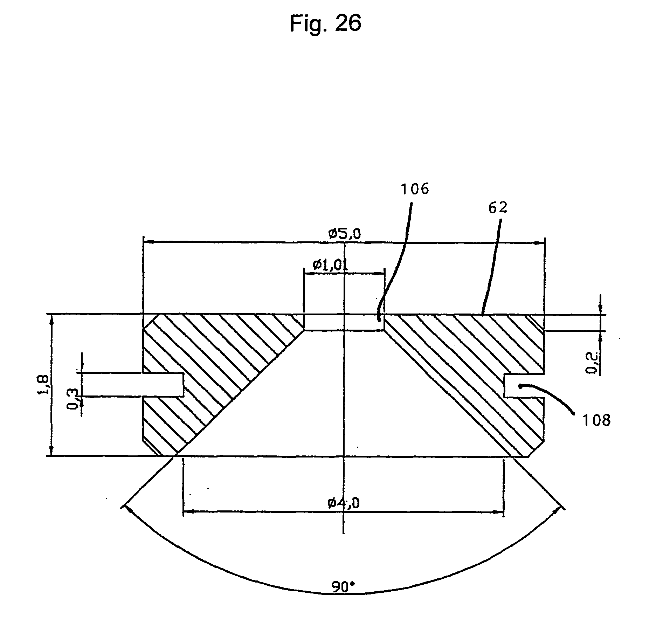

In Fig. 26 ist ein Schnitt in einer axialen Schnittebene durch die Verriegelungsscheibe 62 dargestellt, welche in der Mitte das bereits erwähnte Loch 106 enthält Am Außenumfang weist die Verriegelungscheibe eine Nut 108 auf, in welche die Draht- bzw. Verriegelungsfeder eingreift.

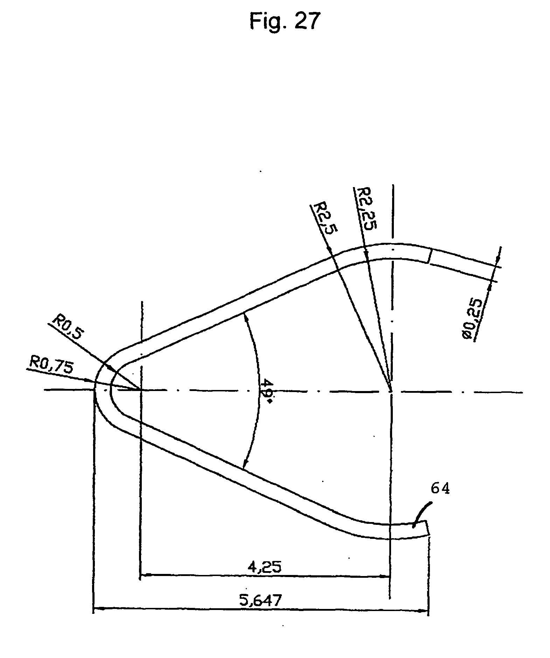

Schließlich zeigt Fig. 27 eine Ansicht der Verriegelungsfeder 64. Es bedarf keiner besonderen Hervorhebung, dass für andere Ausführungsformen der Sperreinrichtung die Abmessungen der Verriegelungsfeder ebenso wie die der übrigen Bauteile abweichend vorgegeben sein können.

**Funktionsbeschreibung Sperreinrichtung**

Die Sperreinrichtung enthält folgende Einzelteile:

- Verriegelungsplättchen      / Verriegelungsscheibe
- Feder Sperreinrichtung      / Verriegelungsfeder
- Verriegelungshalter      / Verriegelungskörper
- Diodenhalter      / Diodenhalter
- Diode
- Kopf R60      / Kopf f. Luerstecker

Die Teile werden wie folgt angeordnet:

Die Diode wird im Diodenhalter eingebracht und mit der ESD Platine von Hinten fixiert. Die Diode sitzt nun im Diodenhalter. Die Diode wird über den Innendurchmesser des Diodenhalters ausgerichtet. Nun kann der Verriegelungshalter auf die Diode aufgeschoben werden. Der Verriegelungshalter wird über den Innendurchmesser des Diodenhalters positioniert. Der O-Ring im Verriegelungshalter fängt leichte Stöße in Längsrichtung ab.

Die Verriegelungsfeder wird in die Nut des Verriegelungsplättchen eingebracht. Diese Anordnung wird in die Öffnung des Verriegelungshalters eingelegt. Der Kopf wird über den Verriegelungshalter und den Diodenhalter geschoben.

Funktionsbeschreibung:

Der Lichtleiter wird über die 1,5 mm Bohrung des Kopfes in den Lichtleitergang eingeführt. Nach ca. 7mm wird der Lichtleiter über eine Verringerung des Durchmessers auf 1,1 mm positioniert. Der Lichtleiter wird dann bis zum Verriegelungsplättchen vorgeschoben.

Das Verriegelungsplättchen wird durch die Vorspannung der Feder auf den Innenrand des Ver riegelungshalters gepresst. Das Plättchen ist dadurch immer dezentriert und verdeckt die 1,01 mm Bohrung des Verriegelungshalters, dadurch kann keine Strahlung austreten.

Der Lichtleiter trifft auf dem Konus des Verriegelungsplättchens auf und drückt dieses entgegen der Federkraft in die Mitte des Verriegelungshalters. Nachdem das Verriegelungsplättchen zentriert wurde, wird die 1,01 mm Bohrung des Verriegelungshalters freigegeben und der Lichtleiter wird durch diese Öffnung in den Einkoppelbereich vorgeschoben. Der Lichtleiter hat den Einkoppelbereich erreicht wenn der Luerstecker den Anschlag am Kopf erreicht hat.

Beim Herausziehen des Lichtleiters schiebt die Verriegelungsfeder das Plättchen wieder in die dezentrale Position, wodurch die 1,01 mm Bohrung wieder verschlossen wird.

Diskussion der Sicherheit des Verriegelungsmechanismus

Der Federdraht der Verriegelungsfeder hat einen Durchmesser von 0,25mm. Die Nut hat 0,3 mm. Da der Draht eine Runde Form besitzt, kann er sich in der Nut nicht verklemmen. Dadurch wird die Sperreinrichtung sicher. Die Kanten des Verriegelungsplättchens sind durch Gleitschleifen auf 0,2 mm gebrochen.

Die Feder presst das Verriegelungsplättchen immer nach außen. Durch die Lage des Lasers im Betrieb ist auch ohne Verriegelungsfeder eine Verriegelung gegeben. Das Verriegelungsplättchen wird durch sein Gewicht nach unten gezogen, wodurch die 1,01 mm Bohrung verschlossen wird. Durch die spezielle Formgebung der Feder kann ein herausspringen der Feder aus der Nut auch ausgeschlossen werden. Die Federform umschließt das Verriegelungsplättchen und sichert es somit:

Eine Neigung des Plättchens hat durch den kleinen Winkel keine Wirkung auf den Verriegelungsmechanismus. Das Plättchen richtet sich beim nächsten Lichtleitereinschub wieder in seine Position aus.

Verschmutzungen des Verriegelungsmechanismus haben keinen Einfuß auf die Funktion der Verriegelung. Es ist nur mit Abriebsstaub des Lichtleiters zu rechnen. Durch die flexible Ausführung des Lichtleiters ist nur mit geringen Abrieb zu rechnen. Bei der jährlichen Überprüfung kann eine Säuberung des Mechanismus einfach durchgeführt werden. Fremdkörper können nur in der Größe von 1,1 mm auftreten, da diese Größe durch die Durchführungsbohrung im Kopf begrenzt wird.

Eventuelles Nachlassen der Federkraft, wird durch Tests nachkontrolliert. Auch Veränderungen durch Fall auf den Boden werden nachgemessen.

**Bezugszeichen**

| | |
|---|---|
| 2 | Lichtleiter |
| 4 | Schutzmantel von 2 |
| 6 | Verbindungskörper / Luerstecker |
| 8 | Lichtleiterende |
| 10 | gebogener Bereich von 2 |
| 12 | Spitze von 2 |
| 14 | Applikationsspritze für den Photosensitizer |
| 16 | Bereich |
| 18 | freies Ende von 2 |

| | |
|---|---|
| 20 | Abstandshalter |
| 22 | angeschliffenes freies Ende von 2 |
| 24 | Spitze von 22 |
| 26 | Lichtquelle / Laserdiode / Halbleiterlaser |
| 28 | Gewindehülse |
| 30 | Kegelteil |
| 32 | Objektivlinse |
| 34 | Linsenhalter |
| 36 | zweite Objektivlinse |
| 38 | Verstellring |
| 40 | Aufnahmekörper |
| 42 | Gehäuserohr / Schutzgehäuse |
| 44 | Batterierohr |
| 46 | Kontaktkappe |
| 48 | Schlüsselhalter |
| 50 | Kopfteil |
| 52 | zentrale Bohrung in 50 |
| 54 | Betriebsanzeige / LEDs |
| 56 | Taste |
| 58 | Verriegelungskörper |
| 60 | Loch in 58 |
| 62 | Verriegelungsscheibe |
| 64 | Verriegelungsfeder / Drahtfeder |
| 66 | Ausnehmung |
| 68 | Diodenhalter |
| 70 | Batterie |
| 72 | Batteriefeder |
| 74 | Steckerbuchse |
| 76 | Buchsenführung |
| 78 | Scheibe |
| 80 | Distanzscheibe |
| 82, 83 | Steckerstift |
| 84 | Etektronikptatine |
| 86 | Tasterfolie |
| 88 | Kugel |
| 90 | hintere Isolierung |
| 92 | Schlagbolzen |
| 94 | vordere Isolierung |
| 96, 97 | O-Ring |
| 98 | Isolierscheibe |
| 100 | Print |
| 102 | Schutzfolie |
| 104 | O-Ring |
| 106 | Loch in 62 |
| 108 | Nut in 62 |

**Patentansprüche**

1. Anordnung zur Reduktion von Mikroorganismen in einem zu therapierenden Bereich, enthaltend ein Bestrahlungsgerät mit einer Lichtquelle (26), sowie zur Therapie eine lichtaktivierbare Substanz, welche auf den zu therapierenden Bereich aufbringbar und von der Lichtquelle (26) bestrahlbar ist, sowie wenigstens einen einen Lichtleiter (2) enthaltenden Applikator, wobei der Applikator einerseits und das Bestrahlungsgerät andererseits mieinander korrespondierende und ineinander greifende Verbindungskörper (6, 50) derart aufweisen, dass das Licht der Lichtquelle (26) durch den Lichtleiter (2) auf den zu therapierenden Bereich strahlt,
**dadurch gekennzeichnet,**
**dass** die Lichtquelle (26) eine Laserdiode ist, der Lichtleiter (2) eine hohe numerische Apertur größer als 0,5 hat und der Lichtleiter (2) eine Lichtaustrittsfläche aufweist, welche mit einer derart vorgegebenen Mikrostruktur versehen ist, dass eine Streuung des Lichtes in eine konische Abstrahlungscharakteristik erreicht wird, durch in axialer Richtung mehr Energie abgegeben wird.

2. Anordnung nach Anspruch 1,
   **dadurch gekennzeichnet,**
   **dass** der Lichtleiter (2) eine hohe numerische Apertur größer als 0,7 aufweist.

3. Anordnung nach Anspruch 1 oder 2,
   **dadurch gekennzeichnet,**
   **dass** das Licht der Lichtquelle (26) direkt in den Lichtleiter (2) des Applikators eingekoppelt wird.

4. Anordnung nach einem der Ansprüche 1 bis 3,
   **dadurch gekennzeichnet,**
   **dass** der Lichtleiter (2) eine definierte Geometrie des Lichtaustrittsbereiches aufweist, welche den Lichtaustritt an die Form der zu belichtenden Areale anpasst und entweder einen flächigen oder einen körperhaften dreidimensionalen Abstrahlbereich erzeugt.

5. Anordnung nach einem der Ansprüche 1 bis 4,
   **dadurch gekennzeichnet,**
   **dass** der Lichtleiter (2) über einen Abstandshalter an der Spitze verfügt, welcher den aktiven Lichtkreis anzeigt und den richtigen Belichtungsabstand festlegt.

6. Anordnung nach einem der Ansprüche 1 bis 5,
   **dadurch gekennzeichnet,**
   **dass** der Lichtleiter (2) eine Geometrie aufweist, die es erlaubt, in enge, komplex geformte Körperhöhlen und Gewebstaschen einzudringen und diese sanft zu öffnen.

7. Anordnung nach einem der Ansprüche 1 bis 6,
   **dadurch gekennzeichnet,**
   **dass** der Lichtleiter (2) eine konische geformte Spitze (12) aufweist.

8. Anordnung nach Anspruch 7,
   **dadurch gekennzeichnet,**
   **dass** die Spitze (12) des Lichtleiters (2) einen Winkel von 1,5 bis 4 Grad zur Achse des Lichtleiters (2) aufweist.

9. Anordnung nach einem der Ansprüche 1 bis 8,
   **dadurch gekennzeichnet,**
   **dass** der Applikator eine feste Abwinkelung zwischen der Spitze (12) des Lichtleiters und der Lichtaustrittrichtung des Verbindungskörpers (4,50) aufweist.

10. Anordnung nach einem der Ansprüche 1 bis 9,
    **dadurch gekennzeichnet,**
    **dass** die vorgegebene Mikrostruktur eine Rauhigkeit von $10\,\mu\text{m}$ bis $200\,\mu\text{m}$ aufweist.

**Claims**

1. An arrangement for reducing micro-organisms in a region to be treated, containing an irradiation device with a light source (26), and also a light-activatable substance for therapy, which can be applied to the region to be treated and can be irradiated by the light source (26), and also at least one applicator containing an optical waveguide (2), wherein the applicator on the one hand and the irradiation device on the other hand have connecting bodies (6, 50) which correspond to one another and engage into one another such that the light of the light source (26) radiates onto the region to be treated by means of the optical waveguide (2),
   **characterised**
   **in that** the light source (26) is a laser diode, the optical waveguide (2) has a high numeric aperture greater than 0.5 and the optical waveguide (2) has a light-emitting surface which is provided with a microstructure provided in such a manner that a scattering of the light in a conical radiation characteristic is achieved, as a result of which more energy is emitted in the axial direction.

**2.** The arrangement according to Claim 1,
**characterised**
**in that** the optical waveguide (2) has a high numeric aperture larger than 0.7.

**3.** The arrangement according to Claim 1 or 2,
**characterised**
**in that** the light of the light source (26) is coupled directly into the optical waveguide (2) of the applicator.

**4.** The arrangement according to one of Claims 1 to 3,
**characterised**
**in that** the optical waveguide (2) has a defined geometry of the light-emitting region, which adjusts the light emission to the shape of the area to be exposed and either creates a flat or corporeal three-dimensional radiation area.

**5.** The arrangement according to one of Claims 1 to 4,
**characterised**
**in that** the optical waveguide (2) has a spacer at the tip which shows the active circle of light and determines the correct exposure distance.

**6.** The arrangement according to one of Claims 1 to 5,
**characterised**
**in that** the optical waveguide (2) has a geometry which allows it to penetrate into narrow complexly shaped body cavities and tissue bursae and to open the same gently.

**7.** The arrangement according to one of Claims 1 to 6,
**characterised**
**in that** the optical waveguide (2) has a conically shaped tip (12).

**8.** The arrangement according to Claim 7,
**characterised**
**in that** the tip (12) of the optical waveguide (2) has an angle of 1.5 to 4 degrees to the axis of the optical waveguide (2).

**9.** The arrangement according to one of Claims 1 to 8,
**characterised**
**in that** the applicator has a fixed angling between the tip (12) of the optical waveguide and the light emission direction of the connecting body (4, 50).

**10.** The arrangement according to one of Claims 1 to 9,
**characterised**
**in that** the predetermined microstructure has a roughness of 10 $\mu$m to 200 $\mu$m.

**Revendications**

**1.** Agencement destiné à réduire des micro-organismes dans une zone faisant l'objet d'une thérapie, contenant un appareil d'irradiation avec une source de lumière (26) et pour la thérapie une substance activable à la lumière, qui peut être appliquée sur la zone faisant l'objet d'une thérapie et peut être irradiée par la source de lumière (26), et au moins un applicateur contenant un guide de lumière (2), l'applicateur d'une part et l'appareil d'irradiation d'autre part présentant des corps de liaison (6, 50) correspondant les uns aux autres et s'engageant les uns dans les autres, de sorte que la lumière de la source de lumière (26) passe à travers le guide de lumière (2) pour arriver sur la zone faisant l'objet d'une thérapie,
**caractérisé**
**en ce que** la source de lumière (26) est une diode laser, le guide de lumière (2) a une ouverture numérique élevée supérieure à 0,5 et le guide de lumière (2) une surface de sortie de lumière, qui est dotée d'une microstructure prédéfinie, de telle sorte qu'une diffusion de la lumière dans une caractéristique de rayonnement conique est obtenue, ce qui permet de délivrer davantage d'énergie dans la direction axiale.

**2.** Agencement selon la revendication 1,
**caractérisé**

**en ce que** le guide de lumière (2) présente une ouverture numérique élevée supérieure à 0,7.

3. Agencement selon la revendication 1 ou 2,
   **caractérisé**
   **en ce que** la lumière de la source de lumière (26) est injectée directement dans le guide de lumière (2) de l'applicateur.

4. Agencement selon l'une des revendications 1 à 3,
   **caractérisé**
   **en ce que** le guide de lumière (2) présente une géométrie définie de la zone de sortie de lumière, qui adapte la sortie de lumière à la forme des zones à irradier et génère une zone de rayonnement plane ou une zone de rayonnement tridimensionnelle physique.

5. Agencement selon l'une des revendications 1 à 4,
   **caractérisé**
   **en ce que** le guide de lumière (2) dispose d'un écarteur sur le sommet, qui affiche le cercle lumineux actif et définit l'espacement d'exposition correct.

6. Agencement selon l'une des revendications 1 à 5,
   **caractérisé**
   **en ce que** le guide de lumière (2) présente une géométrie, qui permet de pénétrer dans des cavités de corps et poches de tissu étroites et formées de façon complexe et de les ouvrir en douceur.

7. Agencement selon l'une des revendications 1 à 6,
   **caractérisé**
   **en ce que** le guide de lumière (2) présente une pointe (12) conique et formée.

8. Agencement selon la revendication 7,
   **caractérisé**
   **en ce que** la pointe (12) du guide de lumière (2) présente un angle de 1,5 à 4 degrés par rapport à l'axe du guide de lumière (2).

9. Agencement selon l'une des revendications 1 à 8,
   **caractérisé**
   **en ce que** l'applicateur présente un coudage fixe entre la pointe (12) du guide de lumière et le sens de sortie de lumière du corps de liaison (4, 50).

10. Agencement selon l'une des revendications 1 à 9,
    **caractérisé**
    **en ce que** la microstructure prédéfinie présente une rugosité de 10 $\mu$m à 200 $\mu$m.

Fig. 1

## Pocketsonde - senkrecht

### Ausgangsleistung 15,5mW

| Durchmesser | 0,1 mm | 0,2 mm |
|---|---|---|
| Abstand von Lichtquelle zu Glasoberfläche | Gemessene Leistung an Messfläche [mW] | Gemessene Leistung an Messfläche [mW] |
| **1 % Methylenblaulösung** | | |
| 0mm | 0,39 | 0 |
| 5mm | 0,38 | 0 |
| 10mm | 0,35 | 0 |
| 15mm | 0,34 | 0 |
| 20mm | 0,33 | 0 |
| 30mm | 0,29 | 0 |
| **0,1% Methylenblaulösung** | | |
| 0mm | 8,3 | 3,3 |
| 5mm | 8,1 | 3,3 |
| 10mm | 8 | 3,3 |
| 15mm | 7,7 | 3,3 |
| 20mm | 7,1 | 3,1 |
| 30mm | 5,9 | 2,6 |

Fig. 2

Fig. 3

14

**Fig. 4**

**Fig. 5**

# Fig. 6

# Fig. 7

## Fig. 8

## Fig. 9

## Fig. 10

## Fig. 11

**Fig. 12**

## Fig. 13

# Fig. 14

# Fig. 15

**Fig. 16**

26

28

32

30

34

36

38

40

# Fig. 17

# Fig. 18

**Applikator A**

**Applikator B**

**Fig. 19**

58

60

64

62

**Fig. 20**

Fig. 21

**58**

**62**

**64**

## Fig. 22

# Fig. 23

Fig. 24

96

92

97

26

104

58

64

101

94

100

98

68

102

62

50

6

2

## Fig. 25

Fig. 26

## Fig. 27

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10049999 A1
- WO 0187416 A1
- EP 0637976 B1
- DE 3918965 A1
- WO 9505214 A
- EP 761257 A2
- WO 0213712 A2